(19) Europäisches Patentamt

European Patent Office

Office européen des brevets

(11) **EP 3 250 193 B1**

(12) **EUROPEAN PATENT SPECIFICATION**

(45) Date of publication and mention
of the grant of the patent:
**08.09.2021 Bulletin 2021/36**

(21) Application number: **16702534.5**

(22) Date of filing: **01.02.2016**

(51) Int Cl.:
*A61K 31/01* $^{(2006.01)}$     *A61P 35/00* $^{(2006.01)}$

(86) International application number:
**PCT/EP2016/052076**

(87) International publication number:
**WO 2016/120490 (04.08.2016 Gazette 2016/31)**

(54) **USE OF A MIXTURE OF MODIFIED GLUCOSE POLYMERS FOR REDUCING TUMOR METASTASIS**

VERWENDUNG EINER MISCHUNG VON MODIFIZIERTEN GLUCOSEPOLYMEREN ZUR REDUZIERUNG VON TUMORMETASTASIERUNG

UTILISATION D'UN MÉLANGE DE POLYMÈRES DE GLUCOSE MODIFIÉS POUR LA RÉDUCTION D'UNE MÉTASTASE TUMORALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.01.2015 EP 15153178**

(43) Date of publication of application:
**06.12.2017 Bulletin 2017/49**

(73) Proprietor: **Fresenius Kabi Deutschland GmbH**
**61352 Bad Homburg (DE)**

(72) Inventors:
• **SUNDERMANN, Bernd**
**61381 Friedrichsdorf (DE)**
• **WALZ, Lars**
**61440 Oberursel (DE)**
• **BAASNER, Silke**
**61137 Schöneck (DE)**
• **NOCKEN, Frank**
**60320 Frankfurt (DE)**
• **MEYER, Christoph**
**61348 Bad Homburg (DE)**

(74) Representative: **Fresenius Kabi Deutschland GmbH**
**Patent Department**
**Borkenberg 14**
**61440 Oberursel (DE)**

(56) References cited:
**WO-A1-02/36167          WO-A1-2012/004008**
**US-A1- 2013 196 949**

• **ROCCONI R P ET AL: "Icodextrin enhances survival in an intraperitoneal ovarian cancer murine model utilizing gene therapy", GYNECOLOGIC ONCOLOGY, ACADEMIC PRESS, LONDON, GB, vol. 103, no. 3, 1 December 2006 (2006-12-01), pages 985-989, XP024922034, ISSN: 0090-8258, DOI: 10.1016/J.YGYNO.2006.06.005 [retrieved on 2006-12-01]**

**Description**

FIELD OF THE INVENTION

The present invention relates to a solution, in particular a pharmaceutically acceptable solution, comprising icodextrin and hydroxyalkyl starch (HAS), wherein the icodextrin is present at a concentration of from 1% to 7.5% (w/v) and wherein the HAS is present at a concentration of from 1% to 15% (w/v). The present invention further relates to the aforesaid pharmaceutically acceptable solution for use as a medicament and for use in preventing metastasis formation and/or relapse by administration to a body cavity of a subject afflicted with cancer. The present invention further relates to a kit comprising icodextrin and HAS in pre-weighed amounts and a pharmaceutically acceptable means of dissolving the same (*i.e.*, a diluents); to a device comprising a pharmaceutically acceptable solution of the present invention and means for administering the same; and to a pharmaceutically acceptable solution comprising icodextrin and hydroxyalkyl starch at a total concentration in the range of from 1% to 20% (w/v), wherein the weight ratio of the icodextrin relative to the hydroxyalkyl starch is in the range of from 0.05:1 to 5:1. The various solutions, kits, and devices described herein are suitable for use in preventing metastasis formation and/or relapse by administration to a body cavity of a subject afflicted with cancer.

BACKGROUND

Polysaccharides derived from starch have been used in medicine, e.g. as volume expanders in plasma substitution, but also in clinical hemodialysis (Sommermeyer et al., 1987, Krankenhauspharmazie, 8(8): 271-278; Weidler et al., 1991, Arzneimittelforschung/Drug Research, 41: 494-498). Frequently, specific forms of hydroxyalkylated starch (HAS), in particular hydroxyethylated starch (HES), are used for this purpose. However, over the years, further potential medical uses of polysaccharides have been described, including those that follow.

Beta-(β)-glucans have been studied in oral and i.v. applications as global immune stimulants, in particular in cancer treatment (A. Weitberg, J Exp Clin Cancer Res, 2008, 27:40; WO 2007/084661) and in a mouse sarcoma model (US Patent No. 4,207,312). It was found that β-glucans have no direct cytotoxic effect, e.g. on cancer cells, but do have immune-stimulatory effects (Chan et al., 2009, J Hematol Oncol, 2:25; WO 2004/030613).

Alpha-(α)-glucans, such as amylose, have not been known for their antineoplastic effects. However, it was found recently that hydroxyalkylated starches have a tumor growth reducing effect when administered intravenously (WO 2013/113747, WO 2013/113496).

DE 40 23 788 A1 describes the use of hydroxyalkyl starch for hyperbaric oxygen therapy of the inner ear and various other conditions. The only example describes the administration of a HES solution comprising an extract of ginkgo biloba to a patient receiving hyperbaric treatment. There is, however, no indication of a therapeutic effect of such a treatment.

There are also several disclosures of the use of solutions comprising polyglucans as carriers for pharmaceutically active compounds. US Patent No. 6,207,654 teaches the use of HES to prevent leakage of serum proteins from capillary endothelial junctions and proposes the use of solutions comprising HES and interleukin-2 to treat viral and bacterial infections with the aim of preventing malignancy. Mohamed et al. describe (EJSO, 2003, 29:261) and review (Surg Oncol Clin N Am, 2003, 12:813) the advantages of isotonic high molecular weight solutions as carriers for intraperitoneal chemotherapy. Also, icodextrin has been described as a constituent of a carrier solution used in intraperitoneal adenoviral oncotherapy in a mouse model, where the solution improved overall survival as compared to PBS (Rocconi et al., Gynecologic Oncology, 2006, 103:985).

Moreover, polyglucans including hydroxyalkylated starches have been proposed for reducing postoperative adhesion formation, see, e.g. Gist et al. (Journal of Investigative Surgery, 1996, 9:369-373), Van den Tol et al. (Surgery, 2005, 137(3):348), US Patent No. 5,807,833, and I. Bekes (Dissertation an der Medizinischen Fakultät der RWTH Aachen (2008): "Adhäsions-und Nidationsprophylaxe nach i.p. Implantation von SCOV.ip-Zellen in SCID-Mäuse mittels Icodextrin, Hyaluronsäure und physiologischer NaCl-Lösung"). The latter document also examined the use of an icodextrin-solution for the prevention of nidation of tumor cells in lesions introduced into the abdominal cavity. No significant effect in comparison to the control (NaCl-solution) was found.

Cancer is still one of the major causes of death, especially in the developed countries. Accordingly, improved treatment and prevention of cancer is still the focus of many research projects. Over the years, methods have been devised for treating primary cancers, which in most cases are at least initially effective, and which in many cases involve surgical intervention. However, depending on the kind of cancer, there is a risk of regrowth of the cancer (relapse) and/or of spread of cancer cells to other sites of the body (metastasis). Accordingly, five-year survival rates vary from e.g. 100% for in situ breast cancer to 25% for ovarian cancer and to rates as low as 6% for pancreatic cancer. Thus, in recent years, the primary focus of much research has shifted from treatment of the primary cancer or at least treatment of the visible tumor to prevention and treatment of metastasis and relapse. These topics appear especially demanding for the cancers of body cavities, especially the abdominal cavity; cancers growing in the abdominal cavity cause symptoms only after they have reached a certain size and therefore often remain in the body for extended periods of time before their removal. As a result, the risk of tumor cell detachment from the tumor, leading to metastasis, is much increased in those tumors. This effect contributes to the often abysmal prognosis of patients diagnosed with such cancers.

An example of a 'surgical intervention' to treat cancer is the peritonectomy, a surgical procedure for peritoneal mesothelioma patients. The peritoneal cavity is the space between the membrane lining the abdominal cavity and the membranes surrounding the organs within the abdomen. The goal of the surgery is to remove the cancerous part of the lining of the abdominal cavity. During a peritonectomy, a cytoreductive surgery may be performed, which aims to remove as much cancerous growth as possible from multiple sites in the abdomen. This procedure, also known as cytoreduction, is a complex procedure that may last 10 to 12 hours. It may even involve the removal of parts of the organs in the proximity, including the bowel, gall bladder, liver, pancreas, spleen and stomach.

Surgical intervention aims to remove the tumor, however it is limited to those cancerous cells that are visible to the eye. Therefore such a surgery (for example, a cytoreductive surgery) may be accompanied by other additional treatments to better penetrate the cancerous cells that are invisible to the naked eye.

For example, a chemotherapy, which is understood as the administration of chemotherapeutic agents with antineoplastic and/or cytotoxic activity, is sometimes administered into the abdominal cavity for direct contact with cancer cells, beginning during surgery and lasting from between 90 minutes to about two weeks. In this combination treatment, the chemotherapy aims to kill any cancer cells that were left behind by the cytoreduction. Hyperthermic intraperitoneal chemotherapy (HIPEC) is another technique that can be used in combination with surgery to treat various gastrointestinal cancers, peritoneal mesotheliomas and ovarian cancers that have spread to the lining of the abdomen.

The combination of cytoreductive (debulking) surgery and HIPEC is a two-step process of surgically removing any visible tumor or cancer, and then delivering heated chemotherapy drugs to the affected area. During the second phase, the patient is connected to a series of catheters and a pumping device that bathes the entire abdominal cavity with the heated chemotherapy drugs for approximately 90 minutes to treat any cancer cells that may remain.

A disadvantage of this method is that handling the equipment requires great care and bears a high risk that the health care personnel will come into contact with the cytotoxic agents used. Also, all the equipment used for bathing the abdomen must be treated and discarded as toxic or hazardous waste because it has been in contact with the cytotoxic agents used in chemotherapy.

Pi et al. (Bull Hunan Med Univ, 1999, 24(6): 1) tested use of hyperthermic peritoneal washing solutions at 45°C to prevent tumor cell nidation in mice. The authors concluded that it could be advantageous to postoperatively wash the abdomen of mice with hypotonic water at 45°C, followed by saline at 45°C and then a hypertonic solution containing dextran 40, also at 45°C. However, these results cannot be transferred to clinical practice easily because treating at 45°C would be deleterious to the normal tissue cells of the patient.

SUMMARY OF THE INVENTION

There is a need to provide compositions and methods to prevent metastasis and relapse of tumors, in particular compositions and methods which can be administered without stressing a subject with the severe side effects that accompany antineoplastic or cytotoxic chemotherapeutic agent or the risk of damaging normal tissue by rinsing it with solutions heated to elevated temperatures. The technical problem is solved by the embodiments characterized in the claims and described below.

Accordingly, the present invention relates to a solution comprising icodextrin and hydroxyalkyl starch (HAS), wherein the icodextrin is present at a concentration of from 1% to 7.5% (w/v) and wherein the HAS is present at a concentration of from 1% to 15% (w/v). A preferred solution comprises 3% to 5% (w/v) icodextrin, and 7.5% to 12.5% (w/v) HES. The ranges stated herein are inclusive. For example, a solution comprising icodextrin from 1% to 7.5% (w/v) can include 1% icodextrin, 7.5% icodextrin, or any range or amount between 1% and 7.5% (w/v). Further, any of the amounts provided can be qualified with the term "about". For example, a solution of the invention can comprise icodextrin from about 1% to about 7.5% (w/v) and HAS from about 1% to about 15%.

By way of example, without being limiting, the following preferred embodiments are within the scope of the present invention.

Embodiment 1: A solution comprising icodextrin and hydroxyalkyl starch (HAS), wherein the icodextrin is present at a concentration of from 1% to 7.5% (w/v) and wherein the HAS is present at a concentration of from 1% to 15% (w/v).

Embodiment 2: A composition of the invention, including the solution of embodiment 1, wherein the HAS is hydroxyethyl starch (HES).

Embodiment 3: A composition of the invention, including the solution of embodiment 1 or 2, wherein the HAS, preferably HES, has a molar substitution (MS) value in the range of from 0.1 to 3, preferably of from 0.2 to 1.3, more preferably of from 0.3 to 0.7.

Embodiment 4: A composition of the invention, including the solution of any one of embodiments 1 to 3, wherein the HAS, preferably HES, has an average molecular weight (Mw) of from 5 to 700 kDa, preferably of from 10 to 300 kDa, more preferably of from 70 to 150 kDa.

Embodiment 5: A composition of the invention, including the solution of any one of embodiments 1 to 4, wherein the icodextrin has an average molecular weight (Mw) of from 5 to 30 kDa, preferably of from 10 to 20 kDa, more preferably, of from 13 to 16 kDa.

Embodiment 6: A composition of the invention, including the solution of any one of embodiments 1 to 5, wherein icodextrin is present at a concentration of from 2% to 5% (w/v) and/or wherein the HAS is present at a concentration of from 5% to 12.5% (w/v).

Embodiment 7: A composition of the invention, including the solution of any one of embodiments 1 to 6, wherein icodextrin is present at a concentration of from 3% to 5% (w/v) and/or wherein the HAS is present at a concentration of from 7.5% to 12.5% (w/v).

Embodiment 8: A composition of the invention, including the solution of any one of embodiments 1 to 7, wherein icodextrin is present at a concentration of 4% $\pm$ 1% (w/v) and wherein the HAS is present at a concentration of 10% $\pm$ 1% (w/v).

Embodiment 9: A composition of the invention, including the solution of any one of embodiments 1 to 8, wherein icodextrin is present at a concentration of 4% $\pm$ 0.5% (w/v) and wherein the HAS is present at a concentration of 10% $\pm$ 0.5% (w/v).

Embodiment 10: A composition of the invention, including the solution of any one of embodiments 1 to 9, wherein the total concentration of icodextrin and HAS is at most 20% (w/v), preferably at most 15%.

Embodiment 11: A composition of the invention, including the solution of any one of embodiments 1 to 10, wherein the total concentration of icodextrin and HAS is from 5% to 20% (w/v), preferably from 7% to 15% (w/v).

Embodiment 12: A composition of the invention, including the solution of any one of embodiments 1 to 11, wherein the solution additionally comprises a salt or salts, preferably NaCl, at a concentration of at least 0.8% (w/v).

Embodiment 13: A composition of the invention, including the solution of any one of embodiments 1 to 12, wherein the solution is a pharmaceutically acceptable solution.

Embodiment 14: A pharmaceutically acceptable solution as described herein (e.g., the solution of embodiment 13) for use as a medicament or in the preparation of a medicament for preventing the formation of metastases or the relapse of a cancer.

Embodiment 15: A pharmaceutically acceptable solution as described herein (e.g., the pharmaceutically acceptable solution of embodiment 13) for use in preventing metastasis formation and/or relapse by administration to a body cavity of a subject afflicted with cancer.

Embodiment 16: A pharmaceutically acceptable solution for use as described herein, e.g., in embodiment 15, wherein the cancer is ovarian cancer, ovarian carcinoma, stomach cancer, lung cancer, pancreatic cancer, bladder cancer, liver cancer, colorectal cancer, or breast cancer. Preferably, the cancer is colon cancer.

Embodiment 17: A pharmaceutically acceptable solution for use as described herein, e.g., in embodiment 15 or 16, wherein the metastasis and/or relapse of the cancer in a body cavity of the subject, preferably the abdominal cavity, is prevented.

Embodiment 18: A pharmaceutically acceptable solution for use as described herein, e.g., in any one of embodiments 15 to 17, wherein the solution is for postoperative administration, for intraoperative administration, and/or for preoperative administration (or is administered in a method of treatment postoperatively, intraoperatively, or preoperatively).

Embodiment 19: Use of a solution as described herein, e.g., a solution of embodiments 1 to 13, in cancer treatment, preferably in preventing metastasis formation and/or relapse of cancer.

Embodiment 20: A kit comprising a icodextrin and HAS in pre-weighed amounts (packaged separately or together) and a pharmaceutically acceptable means of dissolving the same (e.g., a carrier) or a kit comprising a solution comprising icodextrin and HAS, e.g., as described herein.

Embodiment 21: A device comprising a pharmaceutically acceptable solution as described herein, e.g., according to embodiment 13, and means for administering the same.

Embodiment 22: Use of icodextrin and HAS for the manufacture of a pharmaceutical composition for preventing metastasis formation in a subject afflicted with cancer.

Embodiment 23: Use of icodextrin and HAS as described herein, e.g., in the use of embodiment 22, wherein the pharmaceutical composition is a pharmaceutically acceptable solution, preferably a pharmaceutically acceptable solution according to embodiment 13.

Embodiment 24: A method for preventing metastasis formation and/or relapse in a subject afflicted with cancer, comprising administering a pharmaceutically acceptable solution comprising icodextrin at a concentration of from 1% to 7.5% (w/v) and hydroxyalkyl starch (HAS) at a concentration of from 1% to 15 % (w/v) to a body cavity of the subject. The solution is administered for a time and in an amount effective to prevent metastasis formation and/or relapse in the subject.

Embodiment 25: The method of embodiment 24, wherein preventing metastasis formation and/or relapse in a subject is preventing metastasis formation and/or relapse in a body cavity of the subject. The pharmaceutically acceptable solution may be administered to the body cavity and/or may prevent metastasis formation and/or relapse of a cancer therein.

Embodiment 26: A pharmaceutically acceptable solution comprising icodextrin and hydroxyalkyl starch at a total concentration in the range of from 1% to 20% (w/v), wherein the weight ratio of the icodextrin relative to the hydroxyalkyl starch is in the range of from 0.05:1 to 5:1. The solution can be used in preventing metastasis formation and/or relapse by administration to a body cavity of a subject afflicted with cancer.

Embodiment 27: A pharmaceutically acceptable solution as described herein, for example, the solution of embodiment 26, wherein the total concentration of icodextrin and hydroxyalkyl starch is in the range of from 2% to 18% (w/v), preferably in the range of from 5% to 16% (w/v), and more preferably in the range of from 7.5% to 15% (w/v).

Embodiment 28: The pharmaceutically acceptable solution,e.g., for use of embodiment 26 or 27, wherein the weight ratio of icodextrin relative to hydroxyalkyl starch is in the range of from 0.1:1 to 4:1, preferably in the range of from 0.2:1 to 3:1, more preferably in the range of from 0.3:1 to 2:1.

Embodiment 29: The pharmaceutically acceptable solution for use of any one of embodiments 26 to 28, wherein the total concentration of icodextrin and hydroxyalkyl starch is in the range of 14% $\pm$ 1% (w/v) and the weight ratio of the icodextrin relative to the hydroxyalkyl starch is in the range of from 0.3:1 to 0.5:1.

As used herein, the terms "have", "comprise" or "include" or any arbitrary grammatical variations thereof are used in a non-exclusive way. Thus, these terms may refer to a situation in which, besides the feature introduced by these terms, no further features are present in the entity described as well as to a situation in which one or more further features are present. As an example, the expressions "A has B", "A comprises B" and "A includes B" may refer to a situation in which, besides B, no other element is present in A (i.e. a situation in which A solely and exclusively consists of B) as well as to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

Also, as used in the following, the term "about" in connection with a value of a parameter relates to the value including a range $\pm 10\%$, preferably $\pm 5\%$, more preferably $\pm 2\%$, most preferably $\pm 1\%$ of the value. Accordingly, e.g., the expression "about 4 g" preferably corresponds to "4 g $\pm$ 10%", i.e. to values of from 3.6 g to 4.4 g. For clarity, a value and the indicator of variation from that value have the same unit; the variation is denominated in the unit, and this applies to cases where the % sign is the unit. Accordingly, the expression 4% $\pm$ 1% corresponds to values of from 3% to 5%. The term A "essentially consisting of" B relates to A consisting at least to a degree of 90%, preferably 95%, more preferably 98%, and most preferably 99% of B. Where A "consists essentially of" B, A consists of B and any other materials or steps that do not materially affect the basic and novel characteristics of A.

BRIEF DESCRIPTION OF THE DRAWINGS

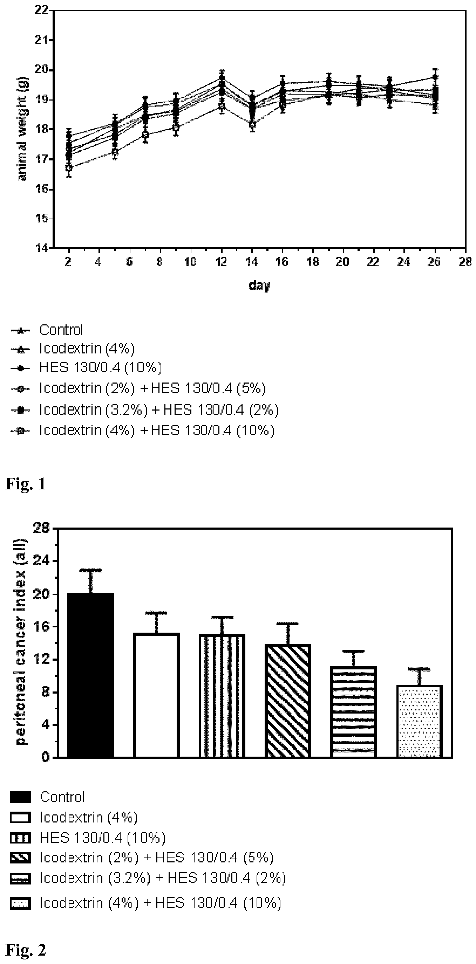

Fig. 1 is a line graph showing the weight of mice inoculated with LS174T tumor cells over time as observed in Example 1. Values on the Y-axis indicate the absolute animal weight in grams $\pm$ the standard error of mean (SEM); values on the X-axis indicate the time in days after the inoculation. The treatments are indicated by the following symbols: "▲" (filled triangle) represents the animals treated with 0.9% isotonic saline (NaCl) as a control; "Δ" unfilled triangle) represents animals treated with an icodextrin solution (4%) (Icodextrin (4%)); "•" (filled circle) represents animals treated with VOLUVEN® blood volume substitute (10%; HES 130/0.4 (10%). "o" (unfilled circle) represents animals treated with a 1:1 (v/v) mixture of an icodextrin solution (4%) and VOLUVEN® blood volume substitute (10%), resulting in a final icodextrin concentration of 2% and a final HES 130/0.4 concentration of 5% (Icodextrin (2%) + HES 130/0.4 (5%)); "■" (filled square) represents animals treated with a 4:1 (v/v) mixture of an icodextrin solution (4%) and VOLUVEN® blood volume substitute (10%), resulting in a final icodextrin concentration of 3.2% and a final HES 130/0.4 concentration of 2% (Icodextrin (3.2%) + HES 130/0.4 (2%)); and "□" (unfilled square) represents animals treated with HES 130/0.4 dissolved at a final concentration of 10% (w/v) in an icodextrin solution (4%) (Icodextrin (4%) + HES 130/0.4 (10%)).

Fig. 2 is a bar graph showing the development of the peritoneal cancer index (PCI; mean of all animals $\pm$ SEM) of mice inoculated with LS174T tumor cells as observed in Example 1. The respective treatments are indicated as follows: the filled bar represents treatment with 0.9% isotonic saline (NaCl) as a control; the unfilled bar represents treatment with an icodextrin (4%) solution, (Icodextrin (4%)); the vertically lined bar represents treatment with VOLUVEN® blood volume substitute (10%) (HES 130/0.4 (10%)); the diagonally striped bar represents treatment with a 1:1 (v/v) mixture of an icodextrin solution (4%) and VOLUVEN® blood volume substitute (10%), resulting in a final icodextrin concentration of 2% and a final HES 130/0.4 concentration of 5% (Icodextrin (2%) + HES 130/0.4 (5%)); the horizontally striped bar represents treatment with a 4:1 (v/v) mixture of an icodextrin solution (4%) and VOLUVEN® blood volume substitute (10%), resulting in a final icodextrin concentration of 3.2% and a final HES 130/0.4 concentration of 2% (Icodextrin (3.2%) + HES 130/0.4 (2%)); and the dotted bar represents treatment with HES 130/0.4 dissolved at a final concentration of 10% (w/v) in an icodextrin solution (4%) (Icodextrin (4%) + HES 130/0.4 (10%)).

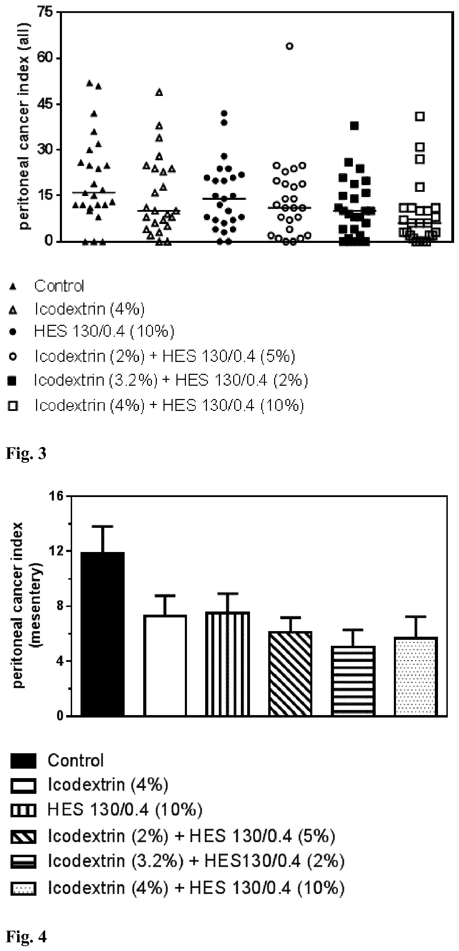

Fig. 3 is a plot showing the development of the peritoneal cancer index (PCI; single values of all animals; black line: median) of mice inoculated with LS174T tumor cells over time as observed in Example 1. The treatments are indicated by the following symbols: "▲" (filled triangle) represents the administration of 0.9% isotonic saline (NaCl) to mice (Control); "Δ" (unfilled triangle) represents administration of an icodextrin solution (4%) to mice (Icodextrin (4%)); "●" (filled circle) represents the administration of VOLUVEN® blood volume substitute (10%) to mice (HES 130/0.4 (10%)); "o" (unfilled circle) represents the administration of a 1:1 (v/v) mixture of an icodextrin solution (4%) and VOLUVEN® blood volume substitute (10%) to mice, resulting in a final icodextrin concentration of 2% and a final HES 130/0.4 concentration of 5% (Icodextrin (2%) + HES 130/0.4 (5%)); "■" (filled square) represents the administration of a 4:1 (v/v) mixture of an icodextrin solution (4%) and VOLUVEN® blood volume substitute (10%) to mice, resulting in a final icodextrin concentration of 3.2% and a final HES 130/0.4 concentration of 2% (Icodextrin (3.2%) + HES 130/0.4 (2%)); and "□" (unfilled square) represents the administration of HES 130/0.4 dissolved at a final concentration of 10% (w/v) in an icodextrin solution (4%) to mice (Icodextrin (4%) + HES 130/0.4 (10%)).

Fig. 4 is a bar graph showing the development of the peritoneal cancer index (PCI; mean of all animals $\pm$ SEM) for the mesentery as observed in Example 1. The respective treatments are indicated as follows: the filled bar represents treatment with 0.9% isotonic saline (NaCl; Control); the filled bar represents treatment with an icodextrin (4%) solution (Icodextrin (4%)); the vertically lined bar represents treatment with VOLUVEN® blood volume substitute (10%), (HES 130/0.4 (10%)); the diagonally striped bar represents treatment with a 1:1 (v/v) mixture of an icodextrin solution (4%) and VOLUVEN® blood volume substitute (10%), resulting in a final icodextrin concentration of 2% icodextrin and a final HES 130/0.4 concentration of 5% (Icodextrin (2%) + HES 130/0.4 (5%)); the horizontally striped bar represents treatment with a 4:1 (v/v) mixture of an icodextrin solution (4%) and VOLUVEN® blood volume substitute (10%), resulting in a final icodextrin concentration of 3.2% and a final HES 130/0.4 concentration of 2% (Icodextrin (3.2%) + HES 130/0.4 (2%)); and the dotted bar represents treatment with HES 130/0.4 dissolved at a final concentration of 10% (w/v) in an icodextrin solution (4%) (Icodextrin (4%) + HES 130/0.4 (10%)).

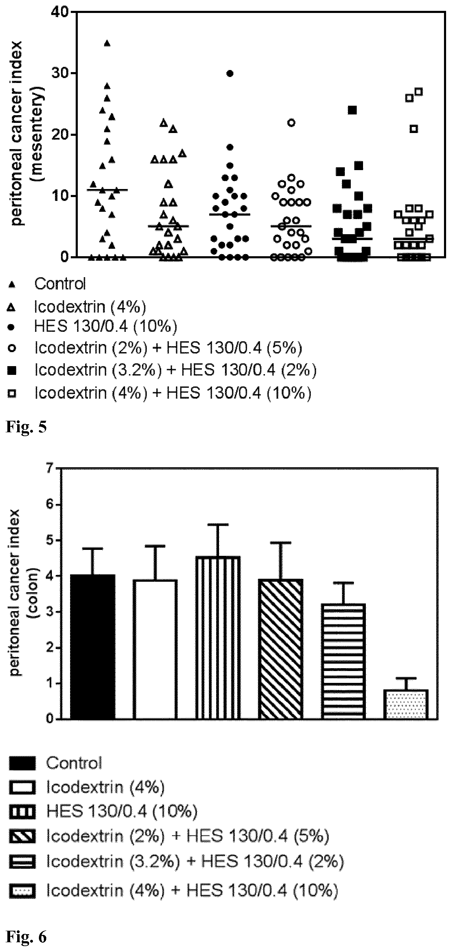

Fig. 5 is a plot showing the development of peritoneal cancer index (PCI; single values of all animals; black line: median) for the mesentery as observed in Example 1. The treatments are indicated by the following symbols: "▲" (filled triangle) represents administration of 0.9% isotonic saline (NaCl) to mice (Control); "△" (unfilled triangle) represents administration of an icodextrin solution (4%) to mice (Icodextrin (4%)); "●" (filled circle) represents administration of a VOLUVEN® blood volume substitute (10%) to mice (HES 130/0.4 (10%)); "o" (unfilled circle) represents the administration of a 1:1 (v/v) mixture of an icodextrin solution (4%) and VOLUVEN® blood volume substitute (10%) to mice, resulting in a final icodextrin concentration of 2% and a final HES 130/0.4 concentration of 5% (Icodextrin (2%) + HES 130/0.4 (5%)); "■" (filled square) represents the administration of a 4:1 (v/v) mixture of an icodextrin solution (4%) and VOLUVEN® blood volume substitute (10%) to mice, resulting in a final icodextrin concentration of 3.2% and a final HES 130/0.4 concentration of 2% (Icodextrin (3.2%) + HES 130/0.4 (2%)); and "□" (unfilled square) represents the administration of HES 130/0.4 dissolved at a final concentration of 10% (w/v) in an icodextrin solution (4%) to mice (Icodextrin (4%) + HES 130/0.4 (10%)).

Fig. 6 is a bar graph showing the development of the peritoneal cancer index (PCI; mean of all animals ± SEM) for the colon as observed in Example 1. The respective treatments are indicated by the following symbols: the filled bar represents treatment with 0.9% isotonic saline (NaCl; Control); the unfilled bar represents treatment with an icodextrin (4%) solution (Icodextrin (4%)); the vertically lined bar represents treatment with VOLUVEN® blood volume substitute (10%; HES 130/0.4 (10%)); the diagonally lined bar represents treatment with a 1:1 (v/v) mixture of an icodextrin solution (4%) and VOLUVEN® blood volume substitute (10%), resulting in a final icodextrin concentration of 2% and a final HES 130/0.4 concentration of 5% (Icodextrin (2%) + HES 130/0.4 (5%)); the horizontally lined bar represents treatment with a 4:1 (v/v) mixture of an icodextrin solution (4%) and VOLUVEN® blood volume substitute (10%), resulting in a final icodextrin concentration of 3.2% and a final HES 130/0.4 concentration of 2% (Icodextrin (3.2%) + HES 130/0.4 (2%)); and the dotted bar represents treatment with HES 130/0.4 dissolved at a final concentration of 10% (w/v) in an icodextrin solution (4%) (Icodextrin (4%) + HES 130/0.4 (10%)).

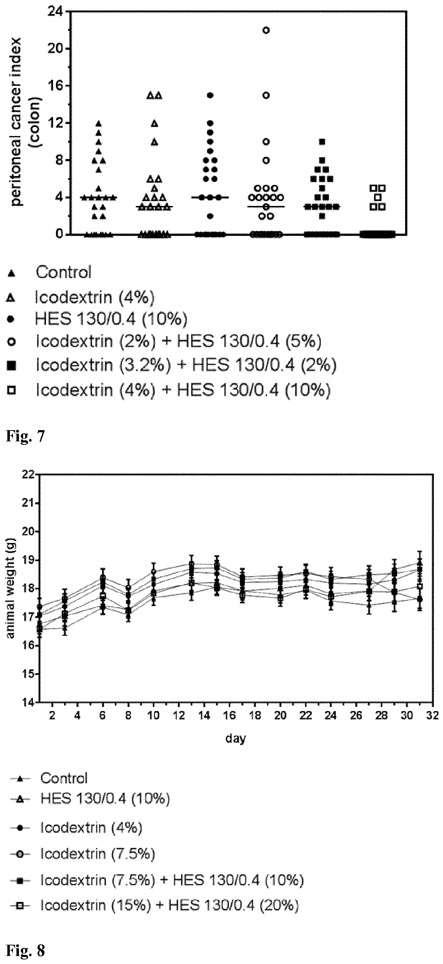

Fig. 7 is a plot showing the development of the peritoneal cancer index (PCI; mean of all animals ± SEM) for the colon as observed in Example 1. The treatments are indicated by the following symbols: "▲" (filled triangle) represents mice administered 0.9% isotonic saline (NaCl; Control); "△" (unfilled triangle) represents mice administered an icodextrin solution (4%; Icodextrin (4%)). The "●" (filled circle) represents mice administered VOLUVEN® blood volume substitute (10%; HES 130/0.4 (10%)). The "o" (unfilled circle) represents mice administered a 1:1 (v/v) mixture of an icodextrin solution (4%) and VOLUVEN® blood volume substitute (10%), resulting in a final icodextrin concentration of 2% and a final HES 130/0.4 concentration of 5% (Icodextrin (2%) + HES 130/0.4 (5%)) . The "■" (filled square) represents mice administered a 4:1 (v/v) mixture of an icodextrin solution (4%) and VOLUVEN® blood volume substitute (10%), resulting in a final icodextrin concentration of 3.2% and a final HES 130/0.4 concentration of 2% (Icodextrin (3.2%) + HES 130/0.4 (2%)). The "□" (unfilled square) represents mice treated with HES 130/0.4 dissolved at a final concentration of 10% (w/v) in an icodextrin solution (4%; Icodextrin (4%) + HES 130/0.4 (10%)).

Fig. 8 is a line graph showing the development of animal weight after mice were inoculated with LS174T tumor cells over time as observed in Example 2. Values on the Y-axis indicate the absolute animal weight in grams ± the standard error of mean (SEM), and values on the X-axis indicate the time in days after the inoculation. The treatments are indicated by the following symbols: the "▲" (filled triangle) represents mice administered a 0.9 % isotonic saline (NaCl) solution (Control); the "△" (unfilled triangle) represents mice administered VOLUVEN® blood volume substitute (10%; HES 130/0.4 (10%); the "●" (filled circle) represents mice treated with an icodextrin solution (4%; Icodextrin (4%; the "o" (unfilled circle) represents mice treated with an icodextrin solution (7.5%; Icodextrin (7.5%)); the "■" (filled square) represents mice treated with HES 130/0.4 (10% final concentration w/v) dissolved in an icodextrin solution (7.5%; Icodextrin (7.5%) + HES 130/0.4 (10%)); the "□" (unfilled square) represents mice treated with Icodextrin (15% final concentration w/v) and HES 130/0.4 (20% final concentration w/v) dissolved in saline for a final concentration of 35% solids (w/v) (Icodextrin (15%) + HES 130/0.4 (20%)).

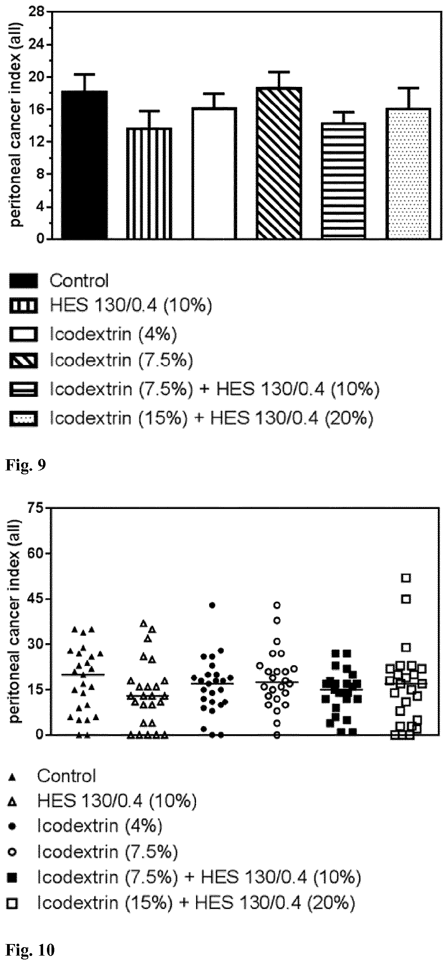

Fig. 9 is a bar graph showing the development of the peritoneal cancer index (PCI; mean of all animals ± SEM) of mice inoculated with LS174T tumor cells as observed in Example 2. The respective treatments are indicated as follows: the filled bar represents mice treated with 0.9 % isotonic saline (NaCl; Control); the vertically lined bar represents mice treated wtih VOLUVEN® blood volume substitute (10%; HES 130/0.4 (10%)); the unfilled bar represents mice treated with icodextrin solution (4%; Icodextrin (4%)); the diagonally lined bar represents mice treated with an icodextrin solution (7.5%; Icodextrin (7.5%)); the horizontally lined bar represents mice treated with HES 130/0.4 (10% final concentration w/v) dissolved in an icodextrin solution (7.5%; Icodextrin (7.5%) + HES 130/0.4 (10%)); and the dotted bar represents mice treated with icodextrin (15% final concentration w/v) and HES 130/0.4 (20% final concentration w/v) dissolved in saline for a final concentration of 35% solids (w/v) (Icodextrin (15%) + HES 130/0.4 (20%)).

Fig. 10 is a plot showing the development of peritoneal cancer index (PCI; single values of all animals; black line: median) of mice inoculated with LS174T tumor cells as observed in example 2. The treatments are indicated by the following symbols: the "A" (filled triangle) represents mice treated with 0.9 % isotonic saline (NaCl; Control); the "Δ" (unfilled triangle) represents mice treated with VOLUVEN® blood volume substitute (10%; HES 130/0.4 (10%); the "●" (filled circle) represents mice treated with an icodextrin solution (4%; Icodextrin (4%)); the "o" (unfilled circle) represents mice treated with an icodextrin solution (7.5%; Icodextrin (7.5%)); the "■" (filled square) represents mice treated wtih HES 130/0.4 (10% final concentration w/v) dissolved in an icodextrin solution (7.5%; Icodextrin (7.5%) + HES 130/0.4 (10%)); the "o" (unfilled square) represents mice treated with icodextrin (15% final concentration w/v) and HES 130/0.4 (20% final concentration w/v) dissolved in saline for a final concentration of 35% solids (w/v) (Icodextrin (15%) + HES 130/0.4 (20%)).

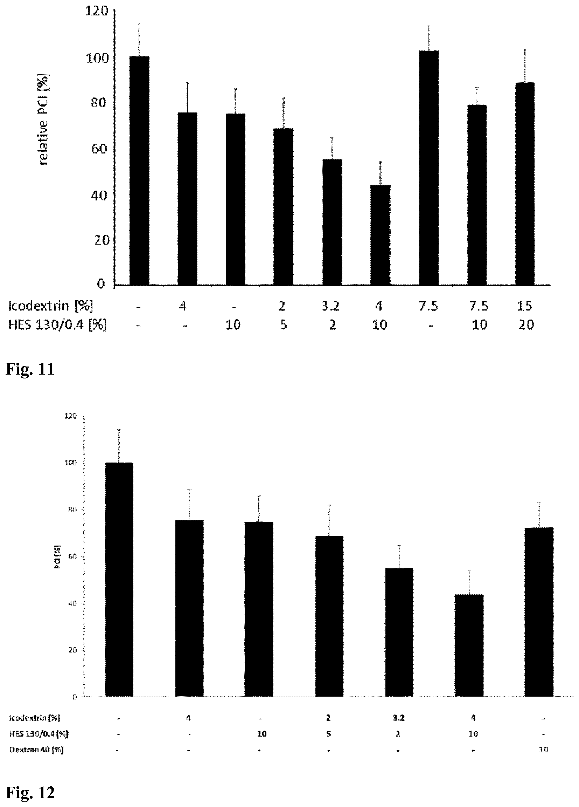

Fig. 11 is a bar graph comparing the peritoneal cancer index (PCI; single values of all animals; over all organs, black line: median) of mice inoculated with LS174T tumor cells as observed in Example 1 and 2. The PCI values are normalized to the respective control group, which is set to 100%. The first six columns are based on data from Example 1 while the data presented in the last three columns stem from Example 2. The treatments are indicated by their concentration of icodextrin and/or HES 130/0.4 in percent (w/v %).

Fig. 12 is a bar graph comparing the peritoneal cancer index (PCI; single values of all animals; black line: median) of mice inoculated with LS174T tumor cells. The PCI values are normalized to the respective control group, which is set to 100%. The first six columns are based on data from Example 1, while the last column shows data from the experiment disclosed in Example 2 in Patent Application No. PCT/EP2014/065990. The treatments are indicated by their concentration of icodextrin and/or HES 130/0.4 or Dextran 40 in w/v %.

DETAILED DESCRIPTION

As will be understood by one of ordinary skill in the art, polysaccharides, in particular biological polysaccharides (biopolymers), consisting of or essentially consisting of anhydroglucose monosaccharide units are referred to as "glucans". Accordingly, e.g., a polysaccharide consisting of or essentially consisting of anhydroglucose units connected via alpha-1,6-glycosidic bonds is referred to as an alpha-(1,6)-glucan.Mutatis mutandis, a polysaccharide consisting of or essentially consisting of anhydroglucose units forming a backbone of alpha-1,4-glycosidically linked molecules with branching points formed by alpha-1,6-glycosidic bonds would be referred to as an alpha-(1,4/1,6)-glucan. It is also understood by one of ordinary skill in the art that the anhydroglucose units in a polysaccharide are often referred to as "glucose units" or "glucose molecules" for simplicity.

Methods for analyzing polysaccharides and, in particular, detecting the presence of, and determining the amount of alpha-glycosidic bonds are known in the art. Preferably, the methods are performed by IR and NMR analysis, such as ID and/or 2D NMR spectroscopy, in particular 1H-NMR, 13C-NMR, HSQC, TOCSY, COSY, NOESY and the like, according to standard protocols. Methods of determining the molecular weight of polysaccharides are known in the art. Mw and Mn of the polysaccharides of the present invention generally are determined according to the following method: The "mean molecular weight" as used in the context of the present invention relates to the weight as determined according to MALLS-GPC (Multiple Angle Laser Light Scattering-Gel Permeation Chromatography). For the determination, 2 Tosoh BioSep GMPWXL columns connected in line (13 $\mu$m particle size, diameter 7.8 mm, length 30 cm, Art.no. 08025) are used as stationary phase. The mobile phase is prepared as follows: In a volumetric flask 3.74 g Na-Acetate*$3H_2O$, 0.344 g $NaN_3$ are dissolved in 800 mL Milli-Q water and 6.9 mL acetic acid anhydride are added and the flask filled up to 1 L. Approximately 10 mg of the polysaccharide are dissolved in 1 mL of the mobile phase and particle filtrated with a syringe filter (0.22 mm, mStarII, CoStar Cambridge, MA) The measurement is carried out at a Flow rate of 0.5 mL/min. As detectors a multiple-angle laser light scattering detector and a refractometer maintained at a constant temperature, connected in series, are used. Astra software (Vers. 5.3.4.14, Wyatt Technology Cooperation) is used to determine the mean Mw and the mean Mn of the sample using a dn/dc of 0.147. The value is determined at $\lambda$=690 nm (solvent NaOAc / H$_2$O / 0.02% NaN$_3$, T=20°C) in accordance to the following literature: W.M. Kulicke, U. Kaiser, D. Schwengers, R. Lemmes, Starch, 1991, 43(10): 392-396 and as described in WO 2012/004007 A1, Example 1.9. However, Mw and Mn values of HAS and HES related to in this specification are values determined according to the method of Sommermeyer et al. (Krankenhauspharmazie,, 1987, 8:271-278) or according to European Pharmacopoeia 7.0, 01/2011:1785, p.984.

Preferably, the polysaccharides of the present invention, i.e. icodextrin and hydroxyalkylated starch, are derivatives of polysaccharides produced or producible by a living organism, more preferably by an organism from the kingdom plantae, in particular by a vascular plant (tracheophyte). Accordingly, a polysaccharide as such may comprise minor impurities, such as, e.g., in the backbone or in the side chains. Preferably, the minor impurities of the polysaccharide constitute less than 10% of the total mass of the polysaccharide, more preferably less than 5% of the total mass of the polysaccharide, still more preferably less than 4%, still more preferably less than 3%, still more preferably less than 2%, still more preferably less than 1 %, still more preferably less than 0.5%, and most preferably, less than 0.1% of the total mass of the polysaccharide.

The term "icodextrin" is, in principle, known to one of ordinary skill in the art and relates to a glucan typically comprising a backbone of alpha-1,4-glycosidically linked anhydroglucose units together with alpha-1,6- linkages as branch points. Icodextrin is a colloid osmotic agent, derived from maltodextrin. Maltodextrin can be enzymatically derived from any starch. Icodextrin is commercially available as aqueous solution either for peritoneal dialysis or in a different concentration for the reduction of post-surgical adhesions (fibrous bands that form between tissues and organs) after gynecological laparoscopic surgery. Preferred icodextrins have a Mw of 5 to 30 kDa, more preferably 10 to 20 kDa, and, most preferably, 13 to 16 kDa. Preferred icodextrins have a Mn of 3 to 10 kDa, more preferably of 4 to 7.5 kDa, and, most preferably, of 5 to 6 kDa. Accordingly, in a preferred embodiment, the icodextrin has an Mw of 13 to 16 kDa and an Mn of 5 to 6 kDa.

Starch is a well-known polysaccharide according to formula $(C_6H_{10}O_5)_n$ which essentially consists of alpha-D glucose units which are coupled via glycosidic linkages. Usually, starch essentially consists of amylose and amylopectin. Amylose consists of linear chains, wherein the glucose units are linked via alpha-1,4-glycosidic linkages. Amylopectin is a highly branched structure with alpha-1,4-glycosidic linkages and alpha-1,6-glycosidic linkages. Native starches from which hydroxyalkyl starches can be prepared include, but are not limited to, cereal starches, legume starches and potato starches. Cereal starches include, but are not limited to, rice starches, wheat starches such as einkorn starches, spelt starches, soft wheat starches, emmer starches, durum wheat starches, or kamut starches, corn starches, rye starches, oat starches, barley starches, triticale starches, spelt starches, and millet starches such as sorghum starches or teff starches. Other sources may be pea, manioc, sweet potato and bananas. Preferred native starches from which hydroxyalkyl starches are prepared have a high content of amylopectin relative to amylose. The amylopectin content of these starches is, for example, at least 70% by weight, preferably at least 75% by weight, more preferably at least 80% by weight, still more preferably at least 85% by weight, still more preferably at least 90% by weight such as up to 95% by weight, up to 96% by weight, up to 97% by weight, up to 98% by weight, up to 99% by weight, or up to 100% by weight. Native starches having an especially high amylopectin content are, for example, suitable potato starches such as waxy potato starches which are preferably extracted from essentially amylose-free potatoes which are either traditionally bred (for example the natural variety Eliane) or genetically modified amylopectin potato varieties, and starches of waxy varieties of cereals such as waxy corn or waxy rice.

Hydroxyalkyl starch (HAS) is an ether derivative of partially hydrolyzed natural starches in which hydroxyl groups in the starch are suitably hydroxyalkylated. Thus, HAS comprises -O-(alkyl-O)$_n$-H groups attached to its anhydroglucose units such that the proton of at least one hydroxyl group is being replaced with the group -(alkyl-O)$_n$-H, with n being of from 1 to 6, preferably of from 1 to 4, more preferably of from 1 to 2, and most preferably 1. As used herein, the term "alkyl group" is understood to comprise a linear or branched functional group or side-chain that consists of saturated hydrocarbons, preferably of a chain length of 2 to 12 carbon atoms. The saturated hydrocarbon can be linear, such as propyl-, butyl-, pentyl-, hexyl-, heptyl-, octyl-, nonyl-, decanyl-, undecanyl- and dodecanyl-residues; or branched, i.e. wherein the carbon backbone splits off in one or more directions, comprising for example isopropyl-, isobutyl-, tert.-butyl, 1-isopentyl-, 2-isopentyl, 3-isopentyl-, neopentyl-groups. Preferably, alkyl is ethyl; accordingly, the group replacing the proton of at least one hydroxyl group of an anhydroglucose unit preferably is -ethyl-OH; thus HAS preferably is hydroxyethyl starch (HES), more preferably HES as specified elsewhere herein.

As a polymer, and owing to the preparation processes, HAS is a polydisperse compound in which the individual hydroxyalkyl starch molecules may differ with respect to the degree of polymerization, the number and the pattern of the branching sites, and the substitution pattern, i.e. the number and/or sites of the hydroxyalkyl groups. Therefore, hydroxyalkyl starch is usually characterized by statistically averaged parameters. These are, generally, molecular weight distribution, the degree of substitution, and the ratio of C2/C6 substitution.

A particular hydroxyalkyl starch solution is, preferably, defined by the average molecular weight with the help of statistical means. In this context, $M_n$ or Mn is calculated as the arithmetic mean depending on the number of molecules and their molecular weight. The number average molecular weight $M_n$ is defined by the following equation:

$$M_n = \Sigma_i\, n_iM_i\, /\, \Sigma_i\, n_i$$

wherein $n_i$ is the number of hydroxyalkyl starch molecules of species i having molar mass $M_i$. Alternatively, the mass distribution can be described by the weight average molecular weight $M_w$ or Mw. The weight average molecular $M_w$ weight is defined by the following equation:

$$M_w = \Sigma_i\, n_iM_i^{2}\, /\, \Sigma_i\, n_iM_i$$

wherein $n_i$ is the number of hydroxyalkyl starch molecules of species i having molar mass $M_i$. According to the present invention, $M_w$ values of HAS, in particular HES, are preferably in the range of from 1 to 2000 kDa, more preferably of from 5 to 700 kDa, more preferably of from 10 to 300 kDa, even more preferably of from 70 to 150 kDa, most preferably 130 kDa.

It is understood by the skilled person that the average molecular weight may be determined according to Sommermeyer et al. (Krankenhauspharmazie, 1987, 8:271-278) or according to European Pharmacopoeia 7.0, 01/2011:1785, p.984. The difference between the two methods is the value of the light scattering value dn/dc used: in the Sommermeyer method, a dn/dc value of 0.135 is used, whereas this value was changed to 0.147+/-0.001 in the Pharmacopoeia method. If not otherwise noted, values of average molecular weights as used herein relate to values as determined with the Sommermeyer method (loc. cit.).

There are two possibilities of describing the substitution degree. The degree of substitution (DS) of hydroxyalkyl starch is described relatively to the portion of substituted glucose monomers with respect to all glucose moieties. The substitution pattern of hydroxyalkyl starch can also be described as the molar substitution (MS), wherein the number of hydroxyalkyl groups per glucose moiety is counted. In the context of the present invention, the substitution pattern of hydroxyalkyl starch is described in terms of MS, which is, preferably, determined according to Sommermeyer et al. (Krankenhauspharmazie 8 (8), 1987, pp 271-278, in particular page 273) or according to European Pharmacopoeia 7.0, 01/2011:1785, p.984. The values of MS correspond to the degradability of the hydroxyalkyl starch by alpha-amylase. Generally, the higher the MS value of the hydroxyalkyl starch, the lower is its respective degradability. The parameter MS can also be determined according to Ying-Che Lee et al., Anal. Chem., 1983, 55:334-338; or K. L. Hodges et al., 1979, Anal. Chem. 51:2171. According to these methods, a known amount of the hydroxyalkyl starch is subjected to ether cleavage in xylene whereby adipinic acid and hydriodic acid are added. The amount of released iodoalkane is subsequently determined via gas chromatography using toluene as an internal standard and iodoalkane calibration solutions as external standards. According to the present invention, MS values are preferably in the range of from 0.1 to 3, more preferably from 0.2 to 1.3, even more preferably from 0.3 to 0.7, most preferably 0.4. If not otherwise noted, values of average molar substitution as used herein relate to values as determined with the Sommermeyer method (loc. cit.).

The further parameter which is referred to as "C2/C6 ratio" describes the ratio of the number of the anhydroglucose units being substituted in C2 position relative to the number of the anhydroglucose units being substituted in C6 position. During the preparation of the hydroxyalkyl starch, the C2/C6 ratio can be influenced via the pH used for the hydroxyalkylation reaction. Generally, the higher the pH, the more hydroxyl groups in C6 position are hydroxyalkylated. The parameter C2/C6 ratio can be determined, for example, according to Sommermeyer et al., Krankenhauspharmazie, 1987, 8(8):271-278, in particular page 273. According to the present invention, typical values of the C2/C6 ratio are in the range of from 2 to 20, preferably of from 2 to 14, more preferably of from 2 to 12.

For practical reasons, the following nomenclature in the identification of different HAS and HES preparations is applied: An abbreviation letter code indicates the kind of modification (e.g. "HES" for hydroxyethyl starch), followed by two numbers, indicating the average molecular weight and the molecular substitution, respectively. Accordingly, "HES 130/0.4" indicates hydroxyethyl starch with an average molecular weight of 130 kDa and an MS of 0.4. It is understood by the skilled person that, since partial hydrolysis as well as substitution of side chains are statistical processes, the values indicated are average values including a certain range. Preferably, the MS values, and the C2/C6 values indicate a range of values $\pm$ 20%, more preferably $\pm$ 10%, most preferably $\pm$ 5%.

Accordingly, preferred embodiments of the HAS of the present invention are HES 70/0.5 and HES 450/0.7, and a most preferred embodiment of the HAS of the present invention is HES 130/0.4.

Concerning the preparation of hydroxyalkyl starch, more particularly of hydroxyethyl starch, reference is made, for example, to Sommermeyer et al., Chromatographia, 1988, 25:167-168; C. Jungheinrich et al., Clin. Pharmacokin., 2005, 44(7), 2005:681-699; J.-M. Mishler IV, Pharmacology of hydroxyethyl starches, Oxford Medical Publications, 2002, 20:1-30.

The term "solution" is known to the skilled person and relates to a composition comprising or consisting of the ingredients as specified herein, dissolved in a liquid carrier. Preferably, the liquid carrier comprises at least 90%, preferably at least 95%, more preferably at least 98% water; thus, the solution, preferably, is an aqueous solution. In a preferred embodiment, the carrier is pure (100%) water. The compositions of the invention can include solutions as described herein that are not naturally occurring.

Preferably, the solution is a pharmaceutically acceptable solution. As used herein, the term "pharmaceutically acceptable solution" relates to a solution as specified herein above, wherein the ingredients, in particular the liquid carrier, are pharmaceutically acceptable in the sense of being compatible with the other ingredients of the formulation and being not deleterious to the recipient thereof. Preferably, the liquid carrier is selected so as not to affect the biological activity of the polysaccharides. Accordingly, the liquid carrier preferably is an isotonic or mildly hypo- or hypertonic solution of non-deleterious ingredients in a suitable solvent. Preferably, the liquid carrier comprises water, more preferably distilled water. More preferably, the liquid carrier is physiological saline solution, phosphate buffered saline solution, cardioplegic solution, Ringer's solution, or Hank's solution. In addition, the pharmaceutically acceptable solution preferably includes other carriers or nontoxic, nontherapeutic, nonimmunogenic stabilizers and the like. Preferably, the pharmaceutically acceptable solution is provided as ready-to-use solution (ready for infusion), e.g. preferably, provided in a bottle or, more preferably, in a bag.

According to a further preferred embodiment, the solution, in particular the pharmaceutically acceptable solution, comprises further ingredients, more preferably pharmaceutically acceptable ingredients as known to the skilled person and as specified, by way of example, herein below. The term "other ingredients" relates, e.g., to sodium chloride, preferably at a physiological concentration, or to other pharmaceutical acceptable additives and/or excipients, including, e.g. one or more magnesium salts, calcium salts, lactates, and the like. Further preferred pharmaceutically acceptable ingredients are excipients, preferably selected from the list consisting of monosaccharides, disaccharides, inorganic salts, antimicrobial agents, antioxidants, surfactants, buffers, acids, bases, and any combination thereof.

Preferred monosaccharides are saccharides, such as fructose, maltose, galactose, glucose, D-mannose, sorbose, and the like; as disaccharides, lactose, sucrose, trehalose, cellobiose, and the like, are mentioned by way of example. Preferred inorganic salts or buffers are citric acid, sodium chloride, potassium chloride, sodium sulfate, potassium nitrate, sodium phosphate monobasic, sodium phosphate dibasic, and any combination thereof. Preferred antimicrobial agents for preventing or detecting microbial growth are benzalkonium chloride, benzethonium chloride, benzyl alcohol, cetylpyridinium chloride, chlorobutanol, phenol, phenylethyl alcohol, phenylmercuric nitrate, thimersol, and any combination thereof. Preferred antioxidants are ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and any combination thereof. Preferred surfactants are polysorbates, or pluronics sorbitan esters; lipids, such as phospholipids and lecithin and other phosphatidylcholines, phosphatidylethanolamines, acids and fatty esters; steroids, such as cholesterol; and chelating agents, such as EDTA or zinc, and any compatible combination thereof. Preferred acids and bases are hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof, and/or sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumarate, and combinations thereof. Other preferred, preferably pharmaceutically acceptable, ingredients include vitamins, micronutrients, antioxidants, and the like. Preferably, the "other ingredients" are galenic ingredients, i.e. ingredients not mediating a pharmaceutical effect related to cancer cells.

Preferably, the pharmaceutically acceptable solution according to the invention does not comprise an interleukin or interferon. More preferably the solution does not comprise any chemotherapeutic agent with antineoplastic or cytotoxic activity. More preferably the solution does not comprise any agents with antineoplastic or cytotoxic activity other than HAS and icodextrin. More preferably, the pharmaceutically acceptable solution comprises the two polysaccharides of the present invention as the sole ingredients preventing metastasis formation and/or relapse. More preferably, the pharmaceutically acceptable solution comprises the two polysaccharides of the present invention as the sole European Medicines Agency (EMA)- and/or Food and Drug Administration (FDA)-approved anti-cancer compounds.

Preferably, the total concentration of the polysaccharides in the solution, preferably the pharmaceutically acceptable solution, is in the range of from 1% to 20% (w/v), more preferably from 2% to 18% (w/v), even more preferably from 5% to 16% (w/v), most preferably in the range of from 7.5% to 15% (w/v), based on the total weight of the polysaccharides of the present invention, i.e. HAS and icodextrin, and on the total volume of the solution. The total concentration herein is determined as the sum of the single concentrations of the different polysaccharides. How the concentration of these is determined is known to the person skilled in the art. The concentrations of the individual polysaccharides in a mixture can be determined using known methods as described in "European Pharmacopoeia 7.0, 01/2011:1785, p. 984" or "B. Wittgren et al., Int. J. Polym. Anal. Charact., 2002, 7(1-2):19-40" in comparison to standards, either prepared by dissolving the individual polysaccharides in the corresponding carrier or by using commercial products containing known concentrations of HES, such as VOLUVEN® blood volume substitute or of icodextrin-like ADEPT® adhesion reduction solution.

Preferred concentrations and concentration ranges for specific embodiments of the present invention are the following: Preferably, the concentration of HAS, in particular HES, in the pharmacologically acceptable solution is in the range of from 1% to 15%, more preferably from 5% to 12.5% (w/v), even more preferably from 7.5% to 12.5% (w/v), and most preferably about 10% (w/v), based on the total volume of the solution. In a preferred embodiment, the concentration of HAS, in particular HES, in the pharmacologically acceptable solution is 10% $\pm$ 1% (w/v) (i.e. in a range of from 9% to 11% (w/v)), more preferably 10% $\pm$ 0.5% (w/v) (i.e. in a range of from 9.5% to 10.5% (w/v)). Preferably, the concentration of icodextrin in the pharmacologically acceptable solution is in the range of from 1% to 7.5% (w/v), more preferably 2% to 6% (w/v), even more preferably 3% to 5%, most preferably about 4% (w/v). In a preferred embodiment, the concentration of icodextrin in the pharmacologically acceptable solution is 4% $\pm$ 1% (w/v) (i.e. in a range of from 3% to 5% (w/v)), more preferably 4% $\pm$ 0.5% (w/v) (i.e. in a range of from 3.5% to 4.5 % (w/v)). A preferred solution comprises 3% to 5% (w/v) icodextrin, and 7.5 to 12.5 % (w/v) HES.

Further preferred compositions and their concentration ranges are shown in Table 1.

Table 1: preferred compositions of the solution, preferably the pharmaceutically acceptable solution, of the present invention; all compositions are, preferably, aqueous solutions:

| Composition No. | Icodextrin [% (w/v)] | HAS [% (w/v)] |
|---|---|---|
| 1 | 2 - 6 | 2 - 15 |
| 2 | 2 - 6 | 5 - 14 |
| 3 | 2 - 6 | 7.5 - 12 |
| 4 | 2 - 6 | 10 |
| 5 | 3 - 5.5 | 2 - 15 |
| 6 | 3 - 5.5 | 5 - 14 |
| 7 | 3 - 5.5 | 7.5 - 12 |
| 8 | 3 - 5.5 | 10 |
| 9 | 3.5 - 5 | 2 - 15 |
| 10 | 3.5 - 5 | 5 - 14 |
| 11 | 3.5 - 5 | 7.5 - 12 |
| 12 | 3.5 - 5 | 10 |
| 13 | 4 | 2 - 15 |
| 14 | 4 | 5 - 14 |
| 15 | 4 | 7.5 - 12 |
| 16 | 4 | 10 |
| 17 | 3-5 | 7.5 - 12.5 |

Preferred pharmaceutically acceptable solutions comprising the polysaccharides of the present invention are, by way of example: HES 130/0.4, 20 g/L and icodextrin, 30 g/L in physiological saline (0.9%); HES 130/0.4, 100 g/L; and icodextrin, 40 g/L in physiological saline (0.9%); and HES 130/0.4, 100 g/L; and icodextrin, 40 g/L in an aqueous solution further comprising sodium chloride 5.4 g/L, sodium lactate 4.5 g/L, calcium chloride 257 mg/L, and magnesium chloride 61 mg/L. The solutions are especially preferred for preoperative, intraoperative, and postoperative administration.

The present invention also relates to the pharmaceutically acceptable solution of the present invention for use as a medicament. Furthermore, the present invention relates to the pharmaceutically acceptable solution of the present invention for use in preventing metastasis formation and/or relapse by administration to a body cavity of a subject afflicted with cancer.

The term "preventing", as used herein, refers to retaining health with respect to the diseases or disorders referred to herein for a certain period of time in a subject. It will be understood that the period of time is dependent on the amount of the composition which has been administered and on individual factors of the subject discussed elsewhere in this specification. It is to be understood that prevention may not be effective in all subjects treated with the composition according to the present invention. However, the term requires that a, preferably statistically significant, portion of subjects of a cohort or population are effectively prevented from suffering from a disease or disorder referred to herein or its accompanying symptoms. Preferably, a cohort or population of subjects is envisaged in this context which normally, i.e.

without preventive measures according to the present invention, would develop a disease or disorder as referred to herein. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99%. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population. The compositions described herein and the methods of their use may also be described as reducing the likelihood that a patient to whom a composition has been administered will experience the metastatic growth of a cancer or, once considered to be cancer-free, the relapse of a cancer (whether of the same type or different from that previously experienced and whether in the same or a different location within the body).

The term "cancer", as used herein, preferably refers to a proliferative disorder or disease of an animal, preferably a human, caused or characterized by the proliferation of cells which have lost susceptibility to normal growth control ("cancer cells"). This uncontrolled growth may be accompanied by intrusion into and destruction of surrounding tissue and possibly spread of cancer cells to other locations in the body (metastasis). It is known to the skilled person that a cancer may reappear after an initial treatment aiming at removal of the solid manifestation of this cancer or aiming at killing any circulating cancer cells thereof. This reappearance is referred to as "relapse". Preferably, the term "cancer" encompasses tumors and any other proliferative disorders. Thus, the term is meant to include all pathological conditions involving malignant cells, irrespective of stage or of invasiveness. The term, preferably, includes solid tumors arising in solid tissues or organs as well as non-solid, e.g. hematopoietic, cancers (e.g. leukemias and lymphomas).

Preferably, according to the invention, the cancer is localized to a specific tissue or organ (e.g. in the ovary, the prostate or the pancreas), and, thus, has not spread beyond the tissue of origin. In another preferred embodiment, the cancer is invasive, and, thus may have spread beyond the layer of tissue in which it originated into the normal surrounding tissues (frequently also referred to as locally advanced cancer). Invasive cancers may or may not be metastatic. Thus, the cancer may be also metastatic. A cancer is metastatic, if it has spread from its original location to distant parts of the body. E.g., it is well known in the art that breast cancer cells may spread to another organ or body part, such as the lymph nodes. Preferably, the cancer is a solid tumor of an organ in fluid communication with at least one body cavity, e.g., preferably, a solid tumor of lung, stomach, pancreas, liver, ovary, uterus, kidney, ileum, colon, rectum, bladder, or prostate. More preferably, the cancer is a solid tumor in or in fluid communication with at least one body cavity as specified elsewhere herein. Preferably, the fluid communication is not fluid communication via blood and/or lymph.

Preferably, the cancer is selected from the list consisting of acute lymphoblastic leukemia (adult), acute lymphoblastic leukemia (childhood), acute myeloidleukemia (adult), acute myeloid leukemia (childhood), adrenocortical carcinoma, adrenocortical carcinoma (childhood), AIDS-related cancers, AIDS-related lymphoma, anal cancer,appendix cancer, astrocytomas (childhood), atypical teratoid/rhabdoid tumor (childhood), central nervous system cancer, basal cell carcinoma, bile duct cancer (extrahepatic), bladder cancer, bladder cancer (childhood), bone cancer, osteosarcoma and malignant fibrous histiocytoma, brain stem glioma (childhood), brain tumor (adult), brain tumor (childhood), brainstem glioma (childhood), central nervous system brain tumor, atypical teratoid/rhabdoid tumor (childhood), brain tumor, central nervous system embryonal tumors (childhood), astrocytomas (childhood) brain tumor, craniopharyngioma, brain tumor (childhood), ependymoblastoma brain tumor (childhood), ependymoma brain tumor (childhood), medulloblastoma brain tumor (childhood), medulloepitheliom brain tumor (childhood), pineal parenchymal tumors of intermediate differentiation, brain tumor (childhood), supratentorial primitive neuroectodermal tumors and pineoblastoma brain tumor, (childhood), brain and spinal cord tumors (childhood), breast cancer , breast cancer (childhood), breast cancer (Male), bronchial tumors (childhood), Burkitt lymphoma, carcinoid tumor (childhood), carcinoid tumor, gastrointestinal carcinoma, atypical teratoid/rhabdoid tumor (childhood), central nervous system (CNS) lymphoma, primary cervical cancer, cervical cancer (childhood), childhood cancers, chordoma (childhood), chronic lymphocytic leukemia, chronic myelogenous leukemia, chronic myeloproliferative disorders, colon cancer, colorectal cancer (childhood), craniopharyngioma (childhood), cutaneous Tcell lymphoma, embryonal tumors, endometrial cancer, ependymoblastoma (childhood), ependymoma (childhood), esophageal cancer, esophageal cancer (childhood), esthesioneuroblastoma (childhood), Ewing sarcoma family of tumors, extracranial germ cell tumor (childhood), extragonadal germ cell tumor, extrahepatic bile duct cancer, eye cancer, intraocular melanoma, retinoblastoma, gallbladder cancer, gastric (stomach) cancer, gastric (stomach) cancer (childhood), gastrointestinal carcinoid tumor, gastrointestinal stromal tumor (GIST), gastrointestinal stromal cell tumor (childhood), germ cell tumor, extracranial (childhood), germ cell tumor, extragonadal, germ cell tumor, ovarian cancer, gestational trophoblastic tumor, glioma (adult), glioma (childhood), brain stem cancer, hairy cell leukemia, head and neck cancer, heart cancer (childhood), hepatocellular (liver) cancer (adult) (primary), hepatocellular (liver) cancer (childhood) (primary), histiocytosis, Langerhans cell, Hodgkin lymphoma (adult), Hodgkin lymphoma (childhood), hypopharyngeal cancer, intraocular melanoma, islet cell tumors (endocrine pancreas), Kaposi sarcoma, kidney (renal cell) cancer, kidney cancer (childhood), Langerhans cell histiocytosis, laryngeal cancer, laryngeal cancer (childhood), leukemia, acute lymphoblastic leukemia (adult), acute lymphoblastic leukemia (childhood), acute myeloid leukemia (adult), acute myeloid leukemia (childhood),, chronic lymphocytic, leukemia, chronic myelogenous, leukemia, lip and oral cavity cancer, liver cancer (adult) (primary), liver cancer (childhood) (primary), non-small cell lung cancer, small cell lung cancer, non-Hodgkin lymphoma, (adult), non-Hodgkin lymphoma, (childhood), primary central nervous system (CNS) lymphoma, Waldenstrom's macroglobulinemia, malignant fibrous histiocytoma of bone and osteosarcoma, medulloblastoma (childhood), medulloepithelioma (childhood), melanoma, intraocular (eye) melanoma, Merkel cell carcinoma, mesothelioma (adult) malignant mesothelioma (childhood), metastatic squamous neck cancer with occult primary, mouth cancer, multiple endocrine neoplasia syndromes (childhood), multiple myeloma/plasma cell neoplasm, mycosis fungoides, myelodysplastic syndromes, myelodysplastic/myeloproliferative neoplasms, myelogenous leukemia, chronic, myeloid leukemia (adult) acute, myeloid leukemia (childhood) acute, multiple myeloma, nasal cavity and paranasal sinus cancer, nasopharyngeal cancer, nasopharyngeal cancer (childhood), neuroblastoma, oral cancer (childhood), lip and oral cavity cancer, oropharyngeal cancer, osteosarcoma and malignant fibrous, histiocytoma of bone, ovarian cancer (childhood), ovarian epithelial cancer, ovarian germ cell tumor, ovarian low malignant potential tumor, pancreatic cancer, pancreatic cancer (childhood), pancreatic cancer, islet cell tumors, papillomatosis (childhood), paranasal sinus and nasal cavity cancer, parathyroid cancer, penile cancer, pharyngeal cancer, pineal parenchymal tumors of intermediate differentiation (childhood), pineoblastoma and supratentorial primitive neuroectodermal tumors (childhood), pituitary tumor, plasma cell neoplasm/multiple myeloma, pleuropulmonary blastoma, pregnancy and breast cancer, primary central nervous system (CNS) lymphoma, prostate cancer, rectal cancer, renal cell (kidney) cancer, renal pelvis and ureter transitional cell cancer, respiratory tract cancer with chromosome 15 changes, retinoblastoma, rhabdomyosarcoma (childhood), salivary gland cancer, salivary gland cancer (childhood), sarcoma, Ewing sarcoma family of tumors, Kaposi sarcoma, soft tissue (adult) sarcoma, soft tissue (childhood) sarcoma, uterine sarcoma, Sézary syndrome, skin cancer (nonmelanoma), skin cancer (childhood), skin cancer (melanoma), Merkel cell skin carcinoma, small cell lung cancer, small intestine cancer, soft tissue sarcoma (adult), soft tissue sarcoma (childhood), squamous cell carcinoma, stomach (gastric) cancer, stomach (gastric) cancer (childhood), supratentorial primitive neuroectodermal tumors (childhood), cutaneous T-cell lymphoma, testicular cancer, testicular cancer (childhood), throat cancer, thymoma and thymic carcinoma, thymoma and thymic carcinoma (childhood), thyroid cancer, thyroid cancer (childhood), transitional cell cancer of the renal pelvis and ureter, gestational trophoblastic tumor, unknown primarysite, carcinoma of adult, unknown primary site, cancer of (childhood), unusual cancers of childhood, ureter and renal pelvis, transitional cell cancer, urethral cancer, uterine cancer, endometrial, uterine sarcoma, vaginal cancer, vaginal cancer (childhood), vulvar cancer, and Wilms tumor.

More preferably, the cancer is a cancer forming a tumor, preferably forming a tumor in a body cavity as specified elsewhere herein. Even more preferably, the cancer is selected from the group comprising abdominal cancer, ovarian cancer, ovarian carcinoma, lung cancer, and bladder cancer, wherein the term abdominal cancer preferably comprises stomach cancer, cancer of the appendix, liver cancer, pancreatic cancer, kidney cancer, peritoneal cancer, peritoneal mesothelioma, adrenocortical cancer and colon cancer.

Even more preferably, the cancer is selected from the group of abdominal cancer and breast cancer, preferably the abdominal cancer is selected from the group of peritoneal cancer and colorectal cancer. The present compositions and methods can be used when the subject's cancer is at an advanced stage.

The term "body cavity", as used herein, relates to any hollow space within the body of a subject which may be filled with liquid, gas, and/or organs or parts thereof, including, e.g. the bladder. Accordingly, the inner lumen of the lymph system and of the circulatory system is not a body cavity. Preferably, the body cavity is a body cavity lined with a serous membrane, e.g. more preferably, an abdominal cavity (cavitas abdominalis), a peritoneal cavity, a pleural cavity, a synovial cavity, a bladder cavity, or a pericardial cavity. Most preferably, the body cavity is the abdominal cavity and the peritoneal cavity. The term "administration to a body cavity" is understood by the skilled person and relates to an administration to the lumen within the body cavity.

The term "subject" relates to an animal, preferably a mammal, more preferably a human.

The term "subject afflicted with cancer" relates to a subject comprising and/or having comprised cancer cells, preferably a tumor, in its body. Preferably, the term relates to a subject for which it is known that it comprises and/or comprised cancer cells; thus, more preferably, the subject afflicted with cancer is a subject diagnosed to suffer from cancer or known to have suffered from cancer.

According to the present invention, the term "administration" relates to application of a composition, preferably of the pharmaceutically acceptable solution, according to the present invention, to a subject. Preferably, the term relates to a continuous administration. More preferably, the term relates to a repeated application, or, most preferably, to a one-time application. The solution according to the invention may be administered into the body cavity and removed and replaced with a second amount of the solution. It is most preferred, however, that the solution is administered as a "one-time-use", i.e. that the solution is administered into the body cavity once and left until it is excreted by the subject's organism.

Preferably, administration relates to administration to a body cavity as specified elsewhere herein, more preferably to the abdominal cavity (cavitas abdominalis), or the peritoneal cavity e.g., preferably, intraperitoneal and/or retroperitoneal administration.

Preferably, administration is performed irrespective of whether the subject received or will receive additional treatment by, e.g. surgical intervention, cytoreductive therapy, and/or chemotherapeutic treatment. Preferably, administration of the solution is used as an additional treatment before, during, or after cytoreductive or loco-regional therapy. Accordingly, the pharmaceutically acceptable solution is preferably for postoperative, intraoperative, and/or preoperative use.

Preferably, administration is administration of the pharmaceutically acceptable solution at a temperature of from 18°C to 43°C. More preferably, administration is administration of the pharmaceutically acceptable solution at a temperature suitable for the patient, i.e. preferably, between ambient temperature (20°C to 25°C) and slightly above body temperature (40°C to 43°C), i.e. from 20°C to 43°C. Preferably, the temperature of the solution is in the range of from 20°C to 42°C, more preferably in the range of from 36°C to 42°C and most preferably in the range of from 36°C to 40°C.

The terms "postoperative", "intraoperative" and "preoperative" administration are known to the skilled person and relate to the timing of administration of the pharmaceutically acceptable solution of the present invention pertaining to a surgical intervention ("surgery"). Preferably, postoperative, intraoperative and preoperative administration is administration in a body cavity of a subject, as specified herein above. It is understood that the term surgery, preferably, relates to any kind of surgical intervention, irrespective whether the surgery is performed in the context of the subject's afflictedness with cancer. More preferably, the surgery of the present invention is a surgical intervention partially or, more preferably, completely removing a tumor, be it a primary or secondary tumor (tumor resection, and/or a metastasis (metastasis resection), preferably from a body cavity, or a surgical intervention for obtaining a biopsy of a tumor and/or a metastasis, preferably from a body cavity. Also preferably, the surgery is cytoreductive surgery as specified elsewhere herein.

The term "postoperative administration", preferably, relates to an administration after a surgical intervention as defined above was performed. Preferably, the term relates to a time frame between immediately after surgery and four weeks thereafter. More preferably, the term relates to a time frame between immediately after surgery and one week thereafter, even more preferably, the term relates to a time frame between immediately after surgery and 24 hours thereafter; most preferably, the term relates to a time frame between immediately after surgery and four hours thereafter. "After surgery" refers to the time after completion of the surgery, i.e. after closure of the previously formed incision, e.g. by sutures or staples. In this case, the polysaccharide or composition of the invention is preferably administered by injection, more preferably by intraperitoneal injection.

The term "intraoperative administration", preferably, relates to an administration during a surgical intervention, i.e. after creating and before closing an incision.

The term "preoperative administration", preferably, relates to an administration in a time frame between four weeks and immediately before surgery, i.e., preferably, before an incision is made. It will be understood that the term, preferably, includes administration at a point in time when a decision if a surgical intervention shall be performed has not yet been made. More preferably, the term relates to a time frame between three weeks and immediately before surgery. Even more preferably, the term relates to a time frame between two weeks and immediately before surgery. Most preferably, the term relates to a time frame between one week and immediately before surgery. It is understood that, preferably, administration of the pharmaceutically acceptable solution of the present invention to a subject will not start before the diagnosis that the subject is afflicted with cancer has been obtained. Thus, preferably, preoperative administration is administration during the time frame between cancer diagnosis and surgery as defined herein above. Preferably, preoperative administration is preoperative administration to a body cavity as specified elsewhere herein.

Advantageously, it was found during the work underlying the present invention that pharmaceutically acceptable solutions comprising the polysaccharides of the present invention, when applied to the abdominal cavity of a mammal, interfere with the settling of cancer cells in the abdominal cavity and that this effect is more pronounced as compared to solutions comprising only one of the polysaccharides. This means that the solutions have an improved utility in the prevention of metastasis and/or relapse whenever there is a risk of settling of free or freely circulating cancer cells, which may arise by detachment from a primary tumor, a metastasis, or a relapse, in a body cavity. By preferably administering a composition that is less toxic than the compositions used in the art (e.g. compositions comprising cytotoxic antineoplastic agents), adverse effects on the patient are reduced, thereby avoiding additional stress on the patient's organism and the risk of contamination of health care personnel when handling the solutions is reduced.

The definitions made above apply *mutatis mutandis* to the following. Additional definitions and explanations made further below also apply for all embodiments described in this specification *mutatis mutandis.*

The present invention also relates to a use of a pharmaceutically acceptable solution as compositionally defined herein above in cancer treatment, preferably for preventing metastasis formation and/or relapse of cancer.

The present invention further relates to a kit comprising icodextrin and HAS in pre-weighed amounts and a pharmaceutically acceptable means of dissolving the same.

The term "kit", as used herein, refers to a collection of the aforementioned compounds, means or reagents of the present invention which may or may not be packaged together. The components of the kit may be comprised by separate vials (i.e. as a kit of separate parts) or provided in a single vial. Moreover, it is to be understood that, preferably, the kit of the present invention is to be used for practicing the methods referred to herein above. It is, more preferably, envisaged that all components are provided in a ready-to-use manner for practicing the methods referred to in this specification. Further, the kit preferably contains instructions for carrying out the the methods. The instructions can be provided by a user's manual in paper- or electronic form. For example, the manual may comprise instructions for handling the equipment required for carrying out the aforementioned methods using the kit of the present invention. Preferably, HAS and icodextrin are comprised in the kit as a mixture.

Further, the present invention relates to a device comprising a pharmaceutically acceptable solution as compositionally defined herein above and means for administering the same.

The term "device", as used herein, relates to a system of means for storing at least the pharmaceutically acceptable solution according the present invention referred to in the claims or herein above, such as pre-filled vials, bottles or bags and a means of administering the same to a subject. Means of administering the pharmaceutically acceptable solution of the present invention are well known to the skilled person and include, e.g. syringes, infusion sets, infusion pumps, and the like. Preferably, the aforesaid means are comprised by a single device.

The present invention further relates to a use of icodextrin and HAS for the manufacture of a pharmaceutical composition for preventing metastasis formation in a subject afflicted with cancer. Preferably, the pharmaceutical composition is a pharmaceutically acceptable solution as specified herein above.

Moreover, the present invention further relates to a method for preventing metastasis formation and/or relapse in a subject afflicted with cancer, comprising

a) administering a pharmaceutically acceptable solution comprising icodextrin at a concentration of from 1% to 7.5% (w/v) and hydroxyalkyl starch (HAS) at a concentration of from 1% to 15% (w/v) to a body cavity of the subject, and
b) thereby preventing metastasis formation and/or relapse in the subject.

The method of the present invention, preferably, is an in vivo method. Preferably, one or more of the steps are performed by automated equipment. Moreover, the method may comprise steps in addition to those explicitly mentioned above. For example, further treatment steps may relate, e.g., to identifying a subject as being afflicted with cancer before step a) or removing the pharmaceutically acceptable solution comprising a polysaccharide from the body cavity after step a), preferably in combination with repeating step a), i.e. flushing repeatedly the body cavity with another dose of the pharmaceutically acceptable solution. Preferably, preventing metastasis formation and/or relapse in a subject is preventing metastasis formation and/or relapse in a body cavity of the subject. More preferably, preventing metastasis formation and/or relapse in a subject is preventing metastasis formation and/or relapse in the subject's body cavity to which the pharmaceutically acceptable solution was applied. Preferably, preventing metastasis formation and/or relapse in a body cavity is preventing metastasis formation and/or relapse in at least one of the organs and/or tissues connected to the lining of the body cavity.

Preferably, surgical removal of cancer cells is performed before, during, or after the step of administering a pharmaceutically acceptable solution comprising the polysaccharides according to the invention to a body cavity of the subject. More preferably, surgical removal of cancer cells is, in a case wherein the cancer forms a solid tumor, removal of at least part of the visible solid tumor of the cancer before or after administering the aqueous solution in step a). Preferably, the visible solid tumor is the primary tumor. More preferably, at least the primary tumor or a part thereof is removed by surgery in such case. Most preferably, at least the primary tumor is removed completely in such case.

Preferably, the amount of pharmaceutically acceptable solution administered is determined by the size and/or the capacity of the body cavity into which the solution is to be administered. It is understood by the skilled person that, in principle, it is preferable to administer a high volume of the pharmaceutically acceptable solution. However, it is also understood that the volume to be administered is naturally limited by the capacity of the body cavity.

An effective dose of the pharmaceutically acceptable solution of the present invention is a dose which prevents metastasis and/or relapse in a subject. Efficacy and toxicity of compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors, preferably in accordance with any one of the above described methods. As is well known in the medical arts, a dosage for any one patient depends upon many factors, including the patient's size, body surface area, age, the particular compound to be administered, sex, time and route of administration, general health, and other drugs being administered concurrently. Efficacy can be monitored by periodic assessment. A typical dose can be, for example, in the range of 250 mL to 2 L pharmaceutically acceptable solution per single administration into the abdominal cavity; however, doses below or above this exemplary range are envisioned, especially considering the aforementioned factors and considering the smaller size of other body cavities, e.g. the pericard. The total amount of the pharmaceutically acceptable solution administered may therefore range from 20 mL up to 10 L, especially if repeated doses are given. It is envisioned that, preferably, the dose is adjusted accordingly. Generally, the regimen as a regular single administration of the pharmaceutical composition should be in the range of 250 mL up to 3 L.

The pharmaceutically acceptable solution referred to herein is administered at least once in order to prevent a disease or condition recited in this specification. However, the pharmaceutically acceptable solution may be administered more than one time, for example every four to seven days for up to several weeks.

The present invention further relates to a pharmaceutically acceptable solution comprising icodextrin and hydroxyalkyl starch at a total concentration in the range of from 1% to 20% (w/v), wherein the weight ratio of the icodextrin relative to the hydroxyalkyl starch is in the range of from 0.05:1 to 5:1, for use in preventing metastasis formation and/or relapse by administration to a body cavity of a subject afflicted with cancer.

The term "total concentration", as used herein, relates to the arithmetic sum of the icodextrin concentration and the HAS concentration in the pharmaceutically acceptable solution of the present invention. As will be appreciated, other polysaccharides potentially present in the solution additionally are, preferably, not included in the calculation of the total concentration.

The "weight ratio" of the icodextrin relative to the hydroxyalkyl starch of the present invention is calculated by dividing the icodextrin concentration by the HAS concentration in the pharmaceutically acceptable solution. Accordingly, if. e.g. icodextrin is present in the solution at a concentration of 4% (w/v), and HAS is present at a concentration of 10% (w/v), the weight ratio is calculated as 4% / 10% = 0.4, which can also be expressed as 0.4:1. Preferably, the weight ratio of the icodextrin relative to the hydroxyalkyl starch is in the range of from 0.05 :1 to 5:1, more preferably in the range of from 0.1:1 to 4:1, still more preferably in the range of from 0.2:1 to 3:1, most preferably in the range of from 0.3:1 to 2:1.

All references cited in this specification are herewith incorporated by reference with respect to their entire disclosure content and the disclosure content specifically mentioned in this specification.

EXAMPLES

The following Examples merely illustrate the invention. They shall not be construed, whatsoever, to limit the scope of the invention.

**Example 1:**

Summary: Adult female BALB/c nude mice were treated with a single i.p. injection of saline, icodextrin (4%), VOLUVEN® blood volume substitute (10%) alone or in combination with a 4% icodextrin solution and a solution containing 10% HES 130/0.4 dissolved in a 4% icodextrin solution after inoculation with human colon adenocarcinoma cells LS174T (ATCC® CL-188™) to determine tumor cell growth and body weight over the course of the experiment.

Substances:

Saline (0.9% NaCl) (Lot 134002, B. Braun Melsungen AG, Melsungen, Germany) was used as Control. The hydroxyethyl starch (HES) containing test item VOLUVEN® blood volume substitute (10%) (Poly(O-2-hydroxyethyl)starch (HES 130/0.4) 100 g/L, NaCl 9 g/L) (Lot 14FC3308) was obtained from Fresenius Kabi Deutschland GmbH (Bad Homburg, Germany) as ready-to-use product. Icodextrin 4% (40 g/L, sodium chloride 5.4 g/L, sodium lactate 4.5 g/L, calcium chloride 257 mg/L, magnesium chloride 61 mg/L) (Lot 11892004, Baxter Deutschland GmbH, Unterschleißheim, Germany) was purchased as ready-to-use solution. HES 130/0.4 (Lot 17123722) was provided by Fresenius Kabi Deutschland GmbH (Bad Homburg, Germany) as solid powder, dissolved in Icodextrin 4% as commercially available (as described above) to a final concentration of 10% w/v and sterile filtered. All solutions were stored at room temperature (<25°C) until use. All solutions were injected under sterile conditions.

Animals:

Adult female BALB/c nude mice (strain CAnN.Cg-Foxnlnu/Crl) (Charles River GmbH, Sulzfeld, Germany) were used in the study. At the start of experiment they were 6-7 weeks of age and had a median body weight between 15 and 20 g. All mice were maintained under strictly controlled and standardized barrier conditions. They were housed - maximum four mice/cage - in individually ventilated cages under following environmental conditions: 22+/- 3°C room temperature, 45 - 65% relative humidity, 12 hours artificial fluorescent light / 12 hours dark. They received autoclaved food and bedding (Ssniff, Soest, Germany) and autoclaved community tap water ad libitum.

Carcinomatosis model:

The study consisted of 6 experimental groups each containing 25 female BALB/c nude mice. On day 0, $2\times10^6$ LS174T cells in 300 µl PBS were administered by intraperitoneal injection into the abdominal cavity of all BALB/c nude mice (Groups 1-6). Freshly prepared cell suspensions were used for each round of implantation, in which 4 animals each of Groups 1-6 were implanted. For the implantation of 25 animals per group, 6 rounds of implantation with freshly prepared cell suspensions for 24 animals (Groups 1-6) were needed. For the sixth and last round of implantation, the freshly prepared cell suspension was used for 30 mice (5 animals x 6 groups) Within 10 to 15 minutes after cell implantation, each mouse of Groups 4-6 received intraperitoneally 500 µl Icodextrin (4%) + VOLUVEN® blood volume substitute (10%) (1:1 v/v) (Group 4), icodextrin (4%) + VOLUVEN® blood volume substitute (10%) (4:1 v/v) (Group 5) or HES 130/0.4 (10% final concentration w/v) dissolved in icodextrin (4%) (Group 6). Animals of Group 1 received 500 µL saline, animals of Group 2 received 500 µL icodextrin (4%) and animals of Group 3 were treated with 500 µL VOLUVEN® blood volume substitute (10%) (see Table 2). All treatments were administered intraperitoneally (i.p.).

Solutions containing 4% icodextrin are based on the commercially available solution thereof, solutions containing VOLUVEN® blood volume substitute (10%) are based on the commercially available solution. Accordingly, the solutions contain slightly different salt concentrations.

Table 2

| Group | Treatment | Administration volume | Animal Number | Final concentration Icodextrin [%] | Final concentration HES [%] |
|-------|-----------|----------------------|---------------|-----------------------------------|----------------------------|
| 1 | Saline | 500 µl/mouse | 25 | 0 | 0 |
| 2 | Icodextrin (4%) | 500 µl/mouse | 25 | 4 | 0 |
| 3 | Voluven® 10% | 500 µl/mouse | 25 | 0 | 10 |
| 4 | Icodextrin (4%) + VOLUVEN® 10% (1: 1 v/v) | 500 µl/mouse | 25 | 2 | 5 |
| 5 | Icodextrin (4%) + VOLUVEN® 10% (4:1 v/v) | 500 µl/mouse | 25 | 3.2 | 2 |
| 6 | HES 130/0.4 (10% final concentration w/v) dissolved in Icodextrin (4%) | 500 µl/mouse | 25 | 4 | 10 |

During the course of the study, several animals out of Groups 1-5 were sacrificed due to ethical reasons (ascites; swelling of the abdominal wall) ahead of schedule and a necropsy performed. On day 27, the study was terminated due to ethical reasons, all remaining animals sacrificed and a necropsy performed. At necropsy, all animals were weighed and killed by cervical dislocation. Animals were macroscopically inspected and a quantification of visible tumors performed by calculating the peritoneal cancer index (PCI).

For this purpose, all tumors of the abdominal cave were categorized via eleven different regions of interest (see Table 3 below) and classified according to the Lesion-Size Score into LS-0 to LS-4 using the tumor diameters, listed in Table 3. Then the number of tumors within the different regions of interest for each Lesion-Size were added up and multiplied with the corresponding factor 0, 1, 2, 3 or 4 for LS-0, LS-1, LS-2, LS-3 and LS-4, respectively, to obtain the Lesion-Size specific PCI values $PCI_{LS0}$ to $PCI_{LS4}$. Finally, these five results were added up in order to get the total Peritoneal Cancer Index ($PCI_{total}$).

Additionally, organ-specific PCI values were calculated for each group. For this purpose, individual PCI values for each region of interest were calculated for each animal as described above, obtaining the organ-specific PCI values $PCI_{RI1}$ to $PCI_{RI11}$. Finally, for each region of interest, $PCI_{RI}$ values for all animals per group were added up and mean and median values determined.

Table 3: Peritoneal Cancer Index (PCI) determination scheme

| Regions of interest (RI) | | Lesion-Size Score (LS) | | | | | RI- specific PCI values |
|---|---|---|---|---|---|---|---|
| | | LS-0 | LS-1 | LS-2 | LS-3 | LS-4 | |
| | | No visible tumors | <2mm tumor diameter | 2-5mm tumor diameter | 5-10mm tumor diameter | >10mm tumor diameter | |
| 1 | Right peritoneum | | | | | | $PCI_{RI1}$ |
| 2 | Left peritoneum | | | | | | $PCI_{RI2}$ |
| 3 | Stomach | | | | | | $PCI_{RI3}$ |
| 4 | Kidney | | | | | | $PCI_{RI4}$ |
| 5 | Intestine | | | | | | $PCI_{RI5}$ |
| 6 | Caecum | | | | | | $PCI_{RI6}$ |
| 7 | Colon | | | | | | $PCI_{RI7}$ |
| 8 | Liver | | | | | | $PCI_{RI8}$ |
| 9 | Spleen | | | | | | $PCI_{RI9}$ |
| 10 | Diaphragm | | | | | | $PCI_{RI10}$ |
| 11 | Mesentery | | | | | | $PCI_{RI11}$ |
| Lesion-Size specific PCI values | | $PCI_{LS0}$ | $PCI_{LS1}$ | $PCI_{LS2}$ | $PCI_{LS3}$ | $PCI_{LS4}$ | $\sum = PCI_{total}$ |

Statistical evaluation: Animal weights, PCI total values per group as well as organ-specific PCI values were analysed using descriptive data analysis (Mean with SEM; Median). In addition, all single values are shown as well over all samples and organ-specific (single values and median). All data analyses were performed using GraphPad Prism 5 from GraphPad Software, Inc., San Diego, USA.

Results:

Treatment with combinations of polysaccharides caused a reduction of the overall peritoneal cancer index (PCI), and in particular of the PCI for the mesentery, compared both to the control group and to treatments with single polysaccharides. For the PCI of the colon, the effect of a solution comprising 4 % Icodextrin and 10 % HES 130/0.4 (indicated as "Icodextrin (4%) + HES 130/0.4 (10%)") was particularly pronounced. No substance related toxicity was detected as shown by the animal weight development.

**Example 2**

Summary: Adult female BALB/c nude mice were treated with a single i.p. injection of saline, Icodextrin (4%), Icodextrin (7.5%), Voluven® 10% alone or different combinations of HES 130/0.4 and Icodextrin after inoculation with human colon adenocarcinoma cells LS174T (ATCC® CL-188™) to determine tumor cell growth and body weight over the course of the experiment.

Substances:

Saline (0.9% NaCl) (Lot 1440030, B. Braun Melsungen AG, Melsungen, Germany) was used as Control. The hydroxyethyl starch (HES) containing test item Voluven® 10% (Poly(O-2-hydroxyethyl)starch (HES 130/0.4) 100 g/L, NaCl 9 g/L) (Lot 14FC3308) was obtained from Fresenius Kabi Deutschland GmbH (Bad Homburg, Germany) as ready-to-use product. Icodextrin 4% (40 g/L, sodium chloride 5.4 g/L, sodium lactate 4.5 g/L, calcium chloride 257 mg/L, magnesium chloride 61 mg/L) (Lot 13892004, Baxter AG, Vienna, Austria) and Icodextrin 7.5% (75 g/L, sodium chloride 5.4 g/L, sodium lactate 4.5 g/L, calcium chloride 257 mg/L, magnesium chloride 61 mg/L) (Lot 14I18G40, Baxter Healthcare Ltd., Thetford, UK) were purchased as ready-to-use solutions. HES 130/0.4 (Lot 17123722) was provided by Fresenius Kabi Deutschland GmbH (Bad Homburg, Germany) as solid powder. Icodextrin powder (Batch 141001SS-05) was provided by Fresenius Kabi Deutschland GmbH (Bad Homburg, Germany) after dialysis and lyophilisation of the original Icodextrin 4% ready-to-use solution (Lot 13892004). This powder was used to produce the solution containing 15% Icodextrin in saline. All solutions were stored at room temperature (<25°C) until use. All solutions were injected under sterile conditions.

Animals:

Adult female BALB/c nude mice (strain CAnN.Cg-Foxnlnu/Crl) (Charles River GmbH, Sulzfeld, Germany) were used in the study. At the start of experiment they were 6-7 weeks of age and had a median body weight between 16 and 18 g.

All mice were maintained under strictly controlled and standardized barrier conditions. They were housed - maximum four mice/cage - in individually ventilated cages under following environmental conditions: 22+/-3°C room temperature, 45 - 65% relative humidity, 12 hours artificial fluorescent light / 12 hours dark. They received autoclaved food and bedding (Ssniff, Soest, Germany) and autoclaved community tap water ad libitum.

Carcinomatosis model:

The study consisted of 6 experimental groups each containing 25 female BALB/c nude mice. On day 0, 2x106 LS174T cells in 300 $\mu$l PBS were administered by intraperitoneal injection into the abdominal cavity of all BALB/c nude mice (Groups 1-6). Freshly prepared cell suspensions were used for each round of implantation, in which 4 animals each of Groups 1-6 were implanted. For the implantation of 25 animals per group, 6 rounds of implantation with freshly prepared cell suspensions for 24 animals (Groups 1-6) were needed. For the sixth and last round of implantation, the freshly prepared cell suspension was used for 30 mice (5 animals x 6 groups) Within 10 to 15 minutes after cell implantation, each mouse received intraperitoneally 500 $\mu$l Voluven® 10% (Group 2), Icodextrin 4% (Group 3), Icodextrin 7.5% (Group 4), HES 130/0.4 (10%) dissolved in Icodextrin 7.5% (Group 5), HES 130/0.4 (20% final concentration w/v) + Icodextrin (15% final concentration w/v) dissolved in Saline (Group 6) or Saline as control (Group 1), respectively (see Table 4). Solutions containing 4% Icodextrin or 7.5% Icodextrin are the commercially available solutions thereof, the solution named Voluven® 10% is the commercially available solution "Voluven® 10%". Accordingly the solutions contain slightly different salt concentrations. The solution containing 15% Icodextrin and 20% HES 130/0.4 was instead manufactured by dissolving 15% w/v of the isolated Icodextrin and 20% of the provided HES 130/0.4 in saline.

Table 4

| Group | Treatment | Administration volume | Route of application | Animal Number |
|---|---|---|---|---|
| 1 | Saline | 500 $\mu$l/mouse | i.p. | 25 |
| 2 | Voluven® 10% | 500 $\mu$l/mouse | i.p. | 25 |
| 3 | Icodextrin 4% | 500 $\mu$l/mouse | i.p. | 25 |
| 4 | Icodextrin (7.5%) | 500 $\mu$l/mouse | i.p. | 25 |
| 5 | HES 130/0.4 (10% w/v) dissolved in Icodextrin 7.5% | 500 $\mu$l/mouse | i.p. | 25 |
| 6 | HES 130/0.4 (20% w/v) + Icodextrin (15% w/v) dissolved in saline (w/v) | 500 $\mu$l/mouse | i.p. | 25 |

During the course of the study, several animals out of Groups 1-5 were sacrificed due to ethical reasons (ascites; swelling of the abdominal wall) ahead of schedule and a necropsy performed. On day 31, the study was terminated due to ethical reasons, all remaining animals sacrificed and a necropsy performed. At necropsy, all animals were weighed and killed by cervical dislocation. Animals were macroscopically inspected and a quantification of visible tumors performed by calculating the peritoneal cancer index (PCI).

For this purpose, all tumors of the abdominal cave were categorized via eleven different regions of interest (see Table 3, experiment 1) and classified according to the Lesion-Size Score into LS-0 to LS-4 using the tumor diameters, listed in Table 3. Then the number of tumors within the different regions of interest for each Lesion-Size were added up and multiplied with the corresponding factor 0, 1, 2, 3 or 4 for LS-0, LS-1, LS-2, LS-3 and LS-4, respectively, to obtain the Lesion-Size specific PCI values $PCI_{LS0}$ to $PCI_{LS4}$. Finally, these five results were added up in order to get the total Peritoneal Cancer Index ($PCI_{total}$).

Additionally, organ-specific PCI values were calculated for each group. For this purpose, individual PCI values for each region of interest were calculated for each animal as described above, obtaining the organ-specific PCI values $PCI_{RI1}$ to $PCI_{RI11}$. Finally, for each region of interest, $PCI_{RI}$ values for all animals per group were added up and mean and median values determined.

Statistical evaluation:

Animal weights, PCI total values per group as well as organ-specific PCI values were analysed using descriptive data analysis (Mean with SEM; Median). In addition, all single values are shown as well over all samples and organ-specific (single values and median). All data analyses were performed using GraphPad Prism 5 from GraphPad Software, Inc., San Diego, USA.

Results:

Treatment with Voluven® 10% or HES 130/0.4 (10%) in Icodextrin 7.5% caused a reduction of the peritoneal cancer index compared to the control group. Treatment with Icodextrin 4% or HES 130/0.4 (20% w/v) + Icodextrin (15% w/v) dissolved in saline caused a smaller reduction of the PCI. Icodextrin 7.5% administration was associated with no reduction of the PCI. No substance related toxicity was detected as shown by the animal weight development.

**Example 3:** Summary

Fig. 11 shows a compilation of the data obtained in Examples 1 and 2. In order to make the data comparable, total PCI values of the controls (saline) were set to 100% and the further values were expressed as % of control. As will be appreciated, solutions comprising icodextrin up to 5 % and HES up to 10 % show improved protection from tumor cell settling, both as compared to the control and to solutions comprising single polysaccharides. A further increase of the icodextrin concentration above 7.5 % and/or of the HES concentration to 20 % does not cause further improvement. Even though the solution containing 7.5% icodextrin doesn't improve the protective effect compared to a 4% icodextrin solution, it is still effective as compared to the administration of the control solution. Without wishing to be bound by theory, it may be concluded from these data that the protective effect is probably not caused by osmotic (hyperoncotic) effects of the solution of the present invention.

**Example 4:** Comparison with Dextran 40

Fig. 12 shows a compilation of the data obtained in example 1 and from a previous experiment as disclosed as "example 2" in patent application PCT/EP2014/065990. In order to make the data comparable, total PCI values of the controls (saline) were set to 100% and the further values were expressed as % of control. As will be appreciated, solutions comprising icodextrin up to 5 % and HES up to 10 % show improved protection from tumor cell settling, both as compared to the control and to solutions comprising single polysaccharides. This effect is more pronounced compared to the effect of Dextran 40.

**Claims**

1. A pharmaceutically acceptable solution comprising icodextrin and hydroxyalkyl starch (HAS), wherein said icodextrin is present at a concentration of from 1 to 7.5 % (w/v) and wherein said HAS is present at a concentration of from 1 to 15 % (w/v) for use in preventing metastasis formation and/or relapse by administration to a body cavity of a subject afflicted with cancer.

2. The pharmaceutically acceptable solution for use of claim 1, wherein said HAS is hydroxyethyl starch (HES).

3. The pharmaceutically acceptable solution for use of claim 1 or 2, wherein said HAS, preferably HES, has a molar substitution (MS) value in the range of from 0.1 to 3.

4. The pharmaceutically acceptable solution for use of any one of claims 1 to 3, wherein said HAS, preferably HES, has an average molecular weight (Mw) of from 70 to 150 kDa.

5. The pharmaceutically acceptable solution for use of any one of claims 1 to 4, wherein said icodextrin has an average molecular weight (Mw) of from 10 to 20 kDa.

6. The pharmaceutically acceptable solution for use of any one of claims 1 to 5, wherein said icodextrin is present at a concentration of from 3 to 5 % (w/v) and/or wherein said HAS is present at a concentration of from 7.5 to 12.5 % (w/v).

7. The pharmaceutically acceptable solution for use of any one of claims 1 to 6, wherein the total concentration of icodextrin and HAS is from 7 to 15 % (w/v).

8. The pharmaceutically acceptable solution for use of any one of claims 1 to 7, wherein the cancer is ovarian cancer, ovarian carcinoma, stomach cancer, lung cancer, pancreas cancer, bladder cancer, liver cancer, colorectal cancer, breast cancer, preferably colon cancer.

9. The pharmaceutically acceptable solution for use of any one of claims 1 to 8, wherein metastasis and/or relapse in a body cavity of said subject, preferably the abdominal cavity, is prevented.

10. The pharmaceutically acceptable solution for use of any one of claims 1 to 9, wherein said solution is for postoperative administration, for intraoperative administration, and/or for preoperative administration.

11. A kit comprising icodextrin and HAS in pre-weighed amounts and a pharmaceutically acceptable means of dissolving the same for use according to any one of claims 1 to 7.

12. A device comprising the pharmaceutically acceptable solution as compositionally defined in any one of claims 1 to 7 and means for administering the same.

13. A pharmaceutically acceptable solution according to any one of the claims 1-10 comprising icodextrin and hydroxyalkyl starch at a total concentration in the range of from 1 to 20 % (w/v), wherein the weight ratio of the icodextrin relative to the hydroxyalkyl starch is in the range of from 0.05 :1 to 5 : 1, for use in preventing metastasis formation and/or relapse by administration to a body cavity of a subject afflicted with cancer.

14. The pharmaceutically acceptable solution according to claim 13 for use according to claim 13, wherein the total concentration of icodextrin and hydroxyalkyl starch is in the range of from 5 to 16 % (w/v).

15. The pharmaceutically acceptable solution according to claim 14 for use according to claim 13, wherein the total concentration of icodextrin and hydroxyalkyl starch is in the range of 14 % $\pm$ 1 % (w/v) and wherein the weight ratio of the icodextrin relative to the hydroxyalkyl starch is in the range of from 0.3 :1 to 0.5 : 1.

**Patentansprüche**

1. Pharmazeutisch verträgliche Lösung, umfassend Icodextrin und Hydroxylalkylstärke (HAS), wobei Icodextrin in einer Konzentration von 1 bis 7,5 % (Gew./Vol.) vorhanden ist und wobei HAS in einer Konzentration von 1 bis 15 % (Gew./Vol.) vorhanden ist, für die Verwendung bei der Prävention der Metastasenbildung und/oder eines Rückfalls durch Verabreichung an einen Körperhohlraum eines Subjekts, das von Krebs befallen ist.

2. Pharmazeutisch verträgliche Lösung für die Verwendung nach Anspruch 1, wobei HAS Hydroxyethylstärke (HES) ist.

3. Pharmazeutisch verträgliche Lösung für die Verwendung nach Anspruch 1 oder 2, wobei HAS, vorzugsweise HES, einen Molarsubstitutionswert (MS-Wert) in dem Bereich von 0,1 bis 3 aufweist.

4. Pharmazeutisch verträgliche Lösung für die Verwendung nach einem der Ansprüche 1 bis 3, wobei HAS, vorzugsweise HES, ein durchschnittliches Molekulargewicht (Mw) von 70 bis 150 kDa aufweist.

5. Pharmazeutisch verträgliche Lösung für die Verwendung nach einem der Ansprüche 1 bis 4, wobei das Icodextrin ein durchschnittliches Molekulargewicht (Mw) von 10 bis 20 kDa aufweist.

6. Pharmazeutisch verträgliche Lösung für die Verwendung nach einem der Ansprüche 1 bis 5, wobei das Icodextrin in einer Konzentration von 3 bis 5 % (Gew./Vol.) vorhanden ist und/oder wobei HAS in einer Konzentration von 7,5 bis 12,5 % (Gew./Vol.) vorhanden ist.

7. Pharmazeutisch verträgliche Lösung für die Verwendung nach einem der Ansprüche 1 bis 6, wobei die Gesamtkonzentration von Icodextrin und HAS 7 bis 15 % (Gew./Vol.) beträgt.

8. Pharmazeutisch verträgliche Lösung für die Verwendung nach einem der Ansprüche 1 bis 7, wobei der Krebs Eierstockkrebs, Eierstockkarzinom, Magenkrebs, Lungenkrebs, Bauchspeicheldrüsenkrebs, Blasenkrebs, Leberkrebs, Colorektalkrebs, Brustkrebs, vorzugsweise Darmkrebs, ist.

9. Pharmazeutisch verträgliche Lösung für die Verwendung nach einem der Ansprüche 1 bis 8, wobei einer Metastasierung und/oder einem Rückfall in einem Körperhohlraum des Subjekts, vorzugsweise der Bauchhöhle, vorgebeugt wird.

10. Pharmazeutisch verträgliche Lösung für die Verwendung nach einem der Ansprüche 1 bis 9, wobei die Lösung zur postoperativen Verabreichung, zur intraoperativen Verabreichung und/oder zur präoperativen Verabreichung ist.

11. Kit, umfassend Icodextrin und HAS in vorgewogenen Mengen und ein pharmazeutisch verträgliches Mittel zum Auflösen davon für die Verwendung nach einem der Ansprüche 1 bis 7.

12. Vorrichtung, umfassend die pharmazeutisch verträgliche Lösung wie zusammensetzerisch nach einem der Ansprüche 1 bis 7 definiert und ein Mittel zum Verabreichen davon.

13. Pharmazeutisch verträgliche Lösung nach einem der Ansprüche 1 bis 10, umfassend Icodextrin und Hydroxylalkylstärke in einer Gesamtkonzentration in dem Bereich von 1 bis 20 % (Gew./Vol.), wobei das Gewichtsverhältnis von Icodextrin zu Hydroxyalkylstärke in dem Bereich von 0,05:1 bis 5:1 liegt, für die Verwendung bei der Prävention der Metastasenbildung und/oder Rückfalls durch Verabreichung an eine Körperhöhe eines Subjekts, das von Krebs befallen ist.

14. Pharmazeutisch verträgliche Lösung nach Anspruch 13 für die Verwendung nach Anspruch 13, wobei die Gesamtkonzentration von Icodextrin und Hydroxylalkylstärke in dem Bereich von 5 bis 16 % (Gew./Vol.) liegt.

15. Pharmazeutisch verträgliche Lösung nach Anspruch 14 für die Verwendung nach Anspruch 13, wobei die Gesamtkonzentration von Icodextrin und Hydroxyalkylstärke in dem Bereich von 14 % $\pm$ 1 % (Gew./Vol.) liegt und wobei das Gewichtsverhältnis von Icodextrin zu Hydroxyalkylstärke in dem Bereich von 0,3:1 bis 0,5:1 liegt.

## Revendications

1. Solution pharmaceutiquement acceptable comprenant de l'icodextrine et de l'hydroxyalkylamidon (HAS), dans laquelle ladite icodextrine est présente à une concentration de 1 à 7,5 % (m/v) et dans laquelle ledit HAS est présent à une concentration de 1 à 15 % (m/v) pour une utilisation dans la prévention de la formation de métastases et/ou de la rechute par administration dans une cavité corporelle d'un sujet atteint d'un cancer.

2. Solution pharmaceutiquement acceptable pour une utilisation selon la revendication 1, dans laquelle ledit HAS est l'hydroxyéthylamidon (HES).

3. Solution pharmaceutiquement acceptable pour une utilisation selon la revendication 1 ou 2, dans lequel ledit HAS, de préférence HES, a une valeur de substitution molaire (SM) dans la plage de 0,1 à 3.

4. Solution pharmaceutiquement acceptable pour une utilisation selon l'une quelconque des revendications 1 à 3, dans lequel ledit HAS, de préférence HES, a une masse moléculaire moyenne (Mm) de 70 à 150 kDa.

5. Solution pharmaceutiquement acceptable pour une utilisation selon l'une quelconque des revendications 1 à 4, dans lequel ladite icodextrine a une masse moléculaire moyenne (Mm) de 10 à 20 kDa.

6. Solution pharmaceutiquement acceptable pour une utilisation selon l'une quelconque des revendications 1 à 5, dans laquelle ladite icodextrine est présente à une concentration de 3 à 5 % (m/v) et/ou dans laquelle ledit HAS est présent à une concentration de 7,5 à 12,5 % (m/v).

**7.** Solution pharmaceutiquement acceptable pour une utilisation selon l'une quelconque des revendications 1 à 6, dans laquelle la concentration totale d'icodextrine et de HAS est de 7 à 15 % (m/v).

**8.** Solution pharmaceutiquement acceptable pour une utilisation selon l'une quelconque des revendications 1 à 7, dans laquelle le cancer est le cancer de l'ovaire, le carcinome ovarien, le cancer de l'estomac, le cancer des poumons, le cancer du pancréas, le cancer de la vessie, le cancer du foie, le cancer colorectal, le cancer du sein, de préférence le cancer du côlon.

**9.** Solution pharmaceutiquement acceptable pour une utilisation selon l'une quelconque des revendications 1 à 8, dans laquelle la métastase et/ou la rechute dans une cavité corporelle dudit sujet, de préférence la cavité abdominale, est évitée.

**10.** Solution pharmaceutiquement acceptable pour une utilisation selon l'une quelconque des revendications 1 à 9, dans laquelle ladite solution est destinée à une administration postopératoire, à une administration peropératoire et/ou à une administration préopératoire.

**11.** Kit comprenant de l'icodextrine et du HAS en quantités prépesées et un moyen pharmaceutiquement acceptable de dissolution de ceux-ci pour une utilisation selon l'une quelconque des revendications 1 à 7.

**12.** Dispositif comprenant la solution pharmaceutiquement acceptable telle que définie en termes de composition dans l'une quelconque des revendications 1 à 7 et un moyen pour l'administration de celle-ci.

**13.** Solution pharmaceutiquement acceptable selon l'une quelconque des revendications 1 à 10 comprenant de l'icodextrine et de l'hydroxyalkylamidon à une concentration totale dans la plage de 1 à 20 % (m/v), dans laquelle le rapport pondéral de l'icodextrine par rapport à l'hydroxyalkylamidon est dans la plage de 0,05:1 à 5:1, pour une utilisation dans la prévention de la formation de métastases et/ou de la rechute par administration dans une cavité corporelle d'un sujet atteint d'un cancer.

**14.** Solution pharmaceutiquement acceptable selon la revendication 13 pour une utilisation selon la revendication 13, dans laquelle la concentration totale d'icodextrine et d'hydroxyalkylamidon est dans la plage de 5 à 16 % (m/v).

**15.** Solution pharmaceutiquement acceptable selon la revendication 14 pour pour une utilisation selon la revendication 13, dans laquelle la concentration totale d'icodextrine et d'hydroxyalkylamidon est dans la plage de 14 % $\pm$ 1 % (m/v) et dans laquelle le rapport pondéral de l'icodextrine par rapport à l'hydroxyalkylamidon est dans la plage de 0,3:1 à 0,5:1.

**Fig. 1**

**Fig. 2**

▲ Control
△ Icodextrin (4%)
● HES 130/0.4 (10%)
○ Icodextrin (2%) + HES 130/0.4 (5%)
■ Icodextrin (3.2%) + HES 130/0.4 (2%)
□ Icodextrin (4%) + HES 130/0.4 (10%)

**Fig. 3**

■ Control
□ Icodextrin (4%)
▥ HES 130/0.4 (10%)
▧ Icodextrin (2%) + HES 130/0.4 (5%)
▤ Icodextrin (3.2%) + HES130/0.4 (2%)
▦ Icodextrin (4%) + HES 130/0.4 (10%)

**Fig. 4**

▲ Control
△ Icodextrin (4%)
● HES 130/0.4 (10%)
○ Icodextrin (2%) + HES 130/0.4 (5%)
■ Icodextrin (3.2%) + HES 130/0.4 (2%)
□ Icodextrin (4%) + HES 130/0.4 (10%)

**Fig. 5**

■ Control
□ Icodextrin (4%)
▥ HES 130/0.4 (10%)
◩ Icodextrin (2%) + HES 130/0.4 (5%)
▤ Icodextrin (3.2%) + HES 130/0.4 (2%)
▧ Icodextrin (4%) + HES 130/0.4 (10%)

**Fig. 6**

▲ Control
△ Icodextrin (4%)
● HES 130/0.4 (10%)
○ Icodextrin (2%) + HES 130/0.4 (5%)
■ Icodextrin (3.2%) + HES 130/0.4 (2%)
□ Icodextrin (4%) + HES 130/0.4 (10%)

**Fig. 7**

⁻▲⁻ Control
⁻△⁻ HES 130/0.4 (10%)
⁻●⁻ Icodextrin (4%)
⁻○⁻ Icodextrin (7.5%)
⁻■⁻ Icodextrin (7.5%) + HES 130/0.4 (10%)
⁻□⁻ Icodextrin (15%) + HES 130/0.4 (20%)

**Fig. 8**

**Fig. 9**

**Fig. 10**

| Icodextrin [%] | - | 4 | - | 2 | 3.2 | 4 | 7.5 | 7.5 | 15 |
| HES 130/0.4 [%] | - | - | 10 | 5 | 2 | 10 | - | 10 | 20 |

**Fig. 11**

| Icodextrin [%] | - | 4 | - | 2 | 3.2 | 4 | - |
| HES 130/0.4 [%] | - | - | 10 | 5 | 2 | 10 | - |
| Dextran 40 [%] | - | - | - | - | - | - | 10 |

**Fig. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007084661 A
- US 4207312 A
- WO 2004030613 A
- WO 2013113747 A
- WO 2013113496 A
- DE 4023788 A1
- US 6207654 B
- US 5807833 A
- EP 2014065990 W
- WO 2012004007 A1

### Non-patent literature cited in the description

- **SOMMERMEYER et al.** *Krankenhauspharmazie,* 1987, vol. 8 (8), 271-278
- **WEIDLER et al.** *Arzneimittelforschung/Drug Research,* 1991, vol. 41, 494-498
- **A. WEITBERG.** *J Exp Clin Cancer Res,* 2008, vol. 27, 40
- **CHAN et al.** *J Hematol Oncol,* 2009, vol. 2, 25
- **MOHAMED et al.** *EJSO,* 2003, vol. 29, 261
- *Surg Oncol Clin N Am,* 2003, vol. 12, 813
- **ROCCONI et al.** *Gynecologic Oncology,* 2006, vol. 103, 985
- **GIST et al.** *Journal of Investigative Surgery,* 1996, vol. 9, 369-373
- **VAN DEN TOL et al.** *Surgery,* 2005, vol. 137 (3), 348
- **I. BEKES.** Adhäsions-und Nidationsprophylaxe nach i.p. Implantation von SCOV.ip-Zellen in SCID-Mäuse mittels Icodextrin, Hyaluronsäure und physiologischer NaCl-Lösung. *Dissertation an der Medizinischen Fakultät der RWTH Aachen,* 2008
- **PI et al.** *Bull Hunan Med Univ,* 1999, vol. 24 (6), 1
- **W.M. KULICKE ; U. KAISER ; D. SCHWENGERS ; R. LEMMES.** *Starch,* 1991, vol. 43 (10), 392-396
- **SOMMERMEYER et al.** *Krankenhauspharmazie,* 1987, vol. 8, 271-278
- *European Pharmacopoeia,* January 2011, vol. 7.0 (1785), 984
- *European Pharmacopoeia,* January 2011, vol. 7.0, 984
- **YING-CHE LEE et al.** *Anal. Chem.,* 1983, vol. 55, 334-338
- **K. L. HODGES et al.** *Anal. Chem.,* 1979, vol. 51, 2171
- **SOMMERMEYER et al.** *Chromatographia,* 1988, vol. 25, 167-168
- **C. JUNGHEINRICH et al.** *Clin. Pharmacokin.,* 2005, vol. 44 (7), 681-699
- **J.-M. MISHLER IV.** Pharmacology of hydroxyethyl starches. Oxford Medical Publications, 2002, vol. 20, 1-30
- **B. WITTGREN et al.** *Int. J. Polym. Anal. Charact.,* 2002, vol. 7 (1-2), 19-40